# EUROPEAN PATENT SPECIFICATION

(11) **EP 4 440 706 B1**
(45) Date of publication and mention of the grant of the patent: **29.04.2026**
(21) Application number: 22823371.4
(22) Date of filing: 29.11.2022
(51) Int. Cl.: A61Q 7/02, A61K 8/63

(54) **COMPOSITIONS FOR PROMOTING HAIR GROWTH**
ZUSAMMENSETZUNGEN ZUR FÖRDERUNG DES HAARWACHSTUMS
COMPOSITION POUR FAVORISER LA CROISSANCE CAPILLAIRE

(30) Priority: 29.11.2021 EP 21211057
(43) Date of publication of application: 09.10.2024
(73) Proprietor: Estetra SRL, 4000 Liège (BE)
(72) Inventor: GERARD, Céline, 4000 Liège (BE); DION, Valérie, 4000 Liège (BE)
(74) Representative: De Clercq & Partners
(86) International application number: PCT/EP2022/083612
(87) International publication number: WO 2023/094690

(56) References cited:
- CREININ MITCHELL D ET AL: "Estetrol-drospirenone combination oral contraceptive: North American phase 3 efficacy and safety results", CONTRACEPTION, GERON-X, INC., LOS ALTOS, CA, US, vol. 104, no. 3, 15 May 2021 (2021-05-15), pages 222 - 228, XP086705234, ISSN: 0010-7824, [retrieved on 20210515], DOI: 10.1016/J.CONTRACEPTION.2021.05.002
- HU HUI-MIN ET AL: "Estrogen Leads to Reversible Hair Cycle Retardation through Inducing Premature Catagen and Maintaining Telogen", PLOS ONE, vol. 7, no. 7, 5 July 2012 (2012-07-05), pages e40124, XP093022735, DOI: 10.1371/journal.pone.0040124
- H KUHL: "Pharmacology of estrogens and progestogens: influence of different routes of administration", CLIMACTERIC, vol. 8, no. sup1, 1 August 2005 (2005-08-01), GB, pages 3 - 63, XP055500517, ISSN: 1369-7137, DOI: 10.1080/13697130500148875

## Description

### Field of the disclosure

The present disclosure relates to compositions for preventing hair loss and/or promoting hair growth, a cosmetic or therapeutic treatment wherein such compositions are used and to the associated formulations or dosage units comprising such compositions.

As further detailed herein, the treatment exhibits statistically significant efficacy combined with a favourable profile for side effects when compared to currently available methods for preventing hair loss or promoting hair growth.

### Background art

With aging, the rate of hair growth declines accompanied by a reduction in hair thickness. Grey hair appears as the result of decreased melanin production within the hair matrix. This effect is aggravated in menopausal women (i.e. perimenopausal and postmenopausal woman) due to the effects of hormonal imbalance, often leading to a negative self-image. The change in hormone balance can have an influence on the skin composition and on the functioning of the hair follicles. Also, the secretions of the sebaceous glands (sebum) provides emollients for the hair and skin and are under the influence of androgen hormones. Sebum secretion declines with age and can be influenced by estrogen levels.

The hair follicle cycle is characterized by a period of follicle growth (anagen), followed by a period of regression and remodeling (catagen), and finally by a resting period (telogen).

Estrogen levels have been related to both hair growth and hair loss. For example, during pregnancy a woman's estrogen levels are higher than normal, which increases hair follicle growth and leading to full, thick hair. In contrast, when the estrogen level decreases such as after a pregnancy or during and after menopause, more hair follicles will enter into a "resting" phase, leading to hair loss causing thinning and even balding patches.

Topical application of compositions comprising estrogens such as estradiol (E2) or estetrol (E4) on the skin has been extensively studied. Reference is for example made to PCT application WO03103685 reporting on the topical application of a cosmetic composition comprising estetrol. Therein it was suggested that some of the effects of applying estrogen on the skin include slowing down of the rate of hair growth, which was seen as a positive thing to avoid e.g. facial hair growth in menopausal women.

In literature, topical application of estrogens has been deemed preferred such as e.g. reported by Hye-Sun Oh and Robert C. Smart (PNAS, Vol. 93, pp. 12525-12530, October 1996) stating that in general, it has been difficult to sort out the action of local vs. systemic factors that regulate the hair follicle cycle. In summary, the results indicate that the hair growth-modulating effects of topically applied E2 and ICI-182780, a pure estrogen receptor antagonist, are occurring locally rather than systemically.

Although oral (systemic) administration of hormone replacement therapies including estrogens is usually not seen as the best option for a cosmetic application towards e.g. hair loss/growth, it is an interesting route since it avoids the need for daily application of e.g. a cream to the skin/scalp, which certain subjects experience to be cumbersome and/or unpleasant. Another advantage is that with systemic use of estrogens at certain doses, menopause-associated symptoms can be alleviated simultaneously. One long standing concern with respect to this form of administration however is to avoid side effects linked to such systemic administration or accumulation of estrogens in the subject. For example, PCT application WO03103685 describes a preference for topical estrogen application for treating the skin. In contrast, the document states that menopausal symptoms should be treated separately by oral or subcutaneous administration. Also, the proposed dosage was too low to suppress symptoms of hypoestrogenism.

Creinin et al. (Contraception, 2021) reports on phase III efficacy and safety results of an estetrol and drospirenone combined oral contraceptive. The authors conclude that the combination is effective and results in a predictable bleeding pattern with low adverse effect rates.

Hu et al. (Plos One, 2012) describes that estrogen leads to reversible hair cycle retardation through inducing premature catagen and maintaining telogen. Notably, the document describes a hair growth suppressive effect of estrogen and a hair cycle arrest associated with a premature onset of catagen and subsequently prolonged telogen.

Finally, Kuhl (Climateric, 2005) is a review directed to the pharmacology of estrogens and progestogens and more particularly the influence of different routes of administration without any reference to estetrol. Kuhl further describes that functions and effects observed for one estrogen may not be readily extrapolated to other estrogens.

That being said, there remains a need for developing new compositions or formulations that overcome some of these identified problems without increasing the risk of adverse effects such as those linked to current estrogen-related therapies.

### Summary of the disclosure

The scope of the invention is limited to the claims appended hereto.

The hair follicle (HF)'s capability to constantly self-renew and undergo repeated cycles relies on the reciprocal interactions that occur between its mesenchymal and epithelial compartments, so-called epithelial-mesenchymal interactions (EMIs). Therefore, successful strategies aiming to promote HF regeneration/stimulation will work best when targeting the combination of relevant cell populations as well as their functioning/signalling and/or migration. The HF mesenchyme is mainly represented by specialized dermal papilla (DP) cells, whereas the epithelial fraction is commonly comprising keratinocytes (KCs) which can be present in different follicular sources, including the bulge, the outer root sheath (ORS) or the hair bulb itself. Such epithelial cells can be derived from epidermal KCs with stem-like features, either residing in the hair follicle itself or present in the skin and that are capable of forming HF epithelium and create EMIs with DP cells, support DP cell growth, promoting HF formation and of sebaceous gland-like structures (Abreu et al., 2021, Stem Cell Research & Therapy volume 12, Article number: 62).

In postnatal life the hair follicles undergo cyclical growth, comprising a resting phase (telogen), a growth phase (anagen) and a regression phase (catagen). During catagen, the epithelial (keratinocytes) cells at the base of the follicle undergo apoptosis, but the DP cells (fibroblast) remain intact and are pulled or migrate upwards, until they come to rest next to the stem cells of the hair follicle bulge. This situation continues in the telogen (resting) phase. In anagen, the cells at the base of the hair follicle start to proliferate, which results in a downward growth of the follicle and envelopment of the DP. Although DP cells themselves are believed to (virtually) not divide, the number of cells in the DP increases during anagen, possibly as a result of replenishment from neighboring cells of the dermal sheath (mesenchymal cells - fibroblast). At the onset of anagen (growth) the DP activates stem cells in the secondary hair germ leading to new downward growth of follicles. During catagen (regression), the hair follicle detaches from the blood supply and follicle cells undergo apoptosis.

Against this background, the present inventors have now surprisingly found that oral administration of estetrol in a specific dosage range can be beneficial for preventing or restoring hair loss related to or caused by menopausal hormonal imbalance and for increasing hair growth in menopausal (peri- or post-menopausal) women. This leads to an improvement in hair texture, hair quality and hair appearance. More particularly, this leads to an improvement in certain parameters such as but not limited to target area hair count ("TAHC") and target area hair width ("TAHW").

It has unexpectedly been shown that clinically relevant concentrations of E4 are reached in the skin after oral administration. Moreover, E4, unlike E2, can act both on epidermal keratinocytes including e.g. bulge, the outer root sheath (ORS) or the hair bulb cells, on dermal fibroblasts such as e.g. dermal papilla (DP) cells and on the sebaceous gland. Estetrol is shown to have several effects that are beneficial for preventing or reducing hair loss, ranging from influencing fibroblast and keratinocyte growth, migration and epithelial-mesenchymal interaction, over increasing follicle activation and sebum secretion. In addition, estetrol has been shown to have an influence on reducing inflammation of the skin, which is linked to healthiness of hair follicles. This opens up opportunities for providing an oral formulation or dosage unit that can be used for preventing or reducing hair loss.

Without wanting to be bound by any theory, it is believed that estetrol (unlike e.g. estradiol) by acting on keratinocytes may promote and/or prolong anagen and hence growth of the hair follicles and/or may delay the onset of catagen by increasing proliferation of keratinocytes and/or reducing apoptosis thereof, thereby reducing hair loss. In addition, by acting also on fibroblasts (i.e. including DP cells), the epithelial-mesenchymal interaction is believed to be improved by estetrol, leading to active DP cells and hair growth.

In the following numbered paragraphs, some particular examples of the disclosure are described.

Aspect 1. An oral dosage unit comprising an estetrol component in an amount equivalent to about 10 mg to about 25 mg of estetrol for use in preventing or treating hair loss, preferably in a daily amount equivalent to about 10 mg to about 25 mg of estetrol.

Aspect 2. Use of an effective amount of an estetrol component in the manufacture of a composition or medicament for preventing or treating hair loss, wherein the estetrol component is used at a daily amount equivalent to about 10 mg to about 25 mg of estetrol.

Aspect 3. Method of preventing or treating hair loss, comprising oral administration (or oral intake) of an estetrol component at a daily amount equivalent to about 10 mg to about 25 mg of estetrol.

Aspect 4. A cosmetic method of preventing or treating hair loss, comprising oral administration (or oral intake) of an estetrol component at a daily amount equivalent to about 10 mg to about 25 mg of estetrol, preferably comprising oral administration of a composition comprising about 10 mg to about 25 mg of an estetrol component.

Aspect 5. The oral dosage unit for use according to aspect 1, the use according to aspect 2, the prevention or treatment method according to aspect 3, or the method according to aspect 4, wherein said prevention or treatment comprises or results in improvement in hair texture, quality and appearance, preferably wherein said prevention or treatment comprises or results in an improvement in target area hair count (TAHC) and/or target area hair width (TAHW).

Aspect 6. The oral dosage unit for use, the use, or the method according to any one of aspects 1 to 5, wherein said hair loss is hormone-related hair loss, more preferably hair loss caused by menopausal hormone deregulation such as estrogen depletion.

Aspect 7. The oral dosage unit for use, the use, or the method according to any one of aspects 1 to 6, wherein the hair loss is caused by a hair disorder in a postmenopausal woman.

Aspect 8. The oral dosage unit for use, the use, or the method according to any one of aspects 1 to 7, wherein said hair loss is female pattern hair loss or female androgenetic alopecia (AGA) (i.e. female-pattern hair loss).

Aspect 9. The oral dosage unit for use, the use, or the method according to any one of aspects 1 to 8, wherein said preventing or treating includes preventing hair loss, reversing hair loss, slowing down hair loss, reducing hair loss, increasing hair growth, or leads to an improvement in hair texture, quality and/or appearance.

Aspect 10. The oral dosage unit for use, the use, or the method according to any one of aspects 1 to 9, wherein said preventing or treating increases keratinocyte function and/or growth, and connected thereto increases function and/or growth, preferably of epidermal keratinocytes (KCs) such as those residing in the bulge, the outer root sheet (ORS), or the hair bulb.

Aspect 11. The oral dosage unit for use, the use, or the method according to any one of aspects 1 to 10, wherein said preventing or treating increases hair follicle mesenchymal fibroblast function and/or growth, and connected thereto increases the function and/or growth of dermal papilla (DP) cells (i.e. a differentiated hair inducing specialised form of fibroblasts).

Aspect 12. The oral dosage unit for use, the use, or the method according to any one of aspects 1 to 11, wherein said preventing or treating implies delaying the onset of catagen or preventing catagen.

Aspect 13. The oral dosage unit for use, the use, or the method according to any one of aspects 1 to 12, wherein said preventing or treating implies promoting and/or prolonging anagen.

Aspect 14. The oral dosage unit for use, the use, or the method according to any one of aspects 1 to 13, wherein said preventing or treating implies restoring and/or promoting the epithelial-mesenchymal interaction between the hair follicle cells.

Aspect 15. The oral dosage unit for use, the use, or the method according to any one of aspects 1 to 14, wherein said preventing or treating is characterised reducing inflammation and/or oxidative stress in hair follicles.

Aspect 16. The oral dosage unit for use, the use, or the method according to any one of aspects 1 to 15, wherein a daily amount equivalent to about 14 mg to about 21 mg of estetrol is administered, preferably an amount of between about 14 mg to about 16 mg or between about 19 mg to about 21 mg of estetrol.

Aspect 17. The oral dosage unit for use, the use, or the method according to any one of aspects 1 to 16, wherein said estetrol component is estetrol or an ester thereof.

Aspect 18. The oral dosage unit for use, the use, or the method according to any one of aspects 1 to 17, wherein said estetrol component is estetrol monohydrate.

Aspect 19. The oral dosage unit for use, the use, or the method according to any one of aspects 1 to 18, wherein no progestogen is comprised in said dosage unit, or wherein no progestogen is used, or co-administered.

Aspect 20. The oral dosage unit for use, the use, or the method according to any one of aspects 1 to 19, wherein a progestogen is present in the oral dosage unit, or wherein a progestogen is used, co-administered, or administrated after treatment with estetrol.

Aspect 21. The oral dosage unit for use, the use, or the method according to aspect 20, wherein said progestogen is selected from the group comprising: progesterone, drospirenone, norethisterone, norethisteron-acetate (NETA), norethindrone, dydrogesterone, levonorgestrel (LNG), etonogestrel, norgestrel, nomegestrol, nomegestrol-acetate (NOMAC), trimegestone, nestorone, dydrogesterone, gestodene, desogestrel, norgestimate, cyproterone acetate, dienogest, and chlormadinone. In a preferred example, said progestogen is selected from the group comprising drospirenone, progesterone, or dydrogesterone.

Aspect 22. The oral dosage unit for use, the use, or the method according to aspect 20 or 21, wherein said progestogen is present in said oral dosage unit in an amount equivalent to from about 0.25 mg to about 4 mg, such as from about 1 mg to about 4 mg, more preferably from about 1 mg to about 3 mg, from about 2.5 mg to 3.5 mg drospirenone, or wherein said progestogen is administered in an amount equivalent to from about 2.5 mg to 3.5 mg drospirenone. In a preferred example, said progestogen is drospirenone.

Aspect 23. The oral dosage unit for use, the use, or the method according to aspect 20 or 21, wherein said progestogen is present in said oral dosage unit in an amount equivalent to from about 1 mg to 20 mg progesterone, or wherein said progestogen is administered in an amount equivalent to from about 5 mg to 10 mg progesterone. In a preferred example, said progestogen is progesterone.

Aspect 24. The oral dosage unit for use, the use, or the method according to aspect 20 or 21, wherein said progestogen is present in said oral dosage unit in an amount equivalent to from about 25 mg to 300 mg dydrogesterone, or wherein said progestogen is administered in an amount equivalent to from about 100 mg to 200 mg dydrogesterone. In a preferred example, said progestogen is dydrogesterone.

Aspect 25. The oral dosage unit for use, the use, or the method according to any one of aspects 1 to 24, wherein a further active ingredient suitable for preventing or treating hair loss is present in the oral dosage unit or wherein a further active ingredient suitable for preventing or treating hair loss is co-administered or administered before or after treatment with estetrol.

Aspect 26. The oral dosage unit for use or the method according to any one of aspects 1 to 25, wherein the composition is formulated for oral, sublingual, buccal, or sublabial administration.

Aspect 27. The oral dosage unit for use or the method according to any one of aspects 1 to 26, wherein the oral dosage unit is formulated to correspond to a daily dosage unit or respectively is administered as a daily dosage unit.

Aspect 28. In any one of the aspects defined herein, said dosage form may be presented as a kit-of-parts containing a packaging unit, e.g. a blister pack, containing the daily oral dosage units comprising the estetrol component. The skilled person will additionally know that each packaging unit, e.g. blister pack, may be numbered or otherwise marked. Each packaging unit may be a sealed blister pack with a cardboard, paperboard, foil plastic backing and enclosed in a suitable cover.

Also envisaged are packaging units such as bottles. The material of the bottle is not particularly limiting. In preferred examples, the bottle is a glass bottle characterised by a colour capable of reducing or preventing degradation of the contents of the bottle by e.g. UV light while maintaining a degree of transparency that allows for visual inspection of the contents of said bottle. Suitable colours include without limitation amber, cobalt, or vintage green.

Aspect 29. In a particular example of the kit-of-parts according to aspect 28, the packaging unit comprises 28 containers or a multitude of 28 containers, such as 2 to 12 times 28 containers.

The above numbered aspects are further described in the following sections and in the appended claims. The subject matter of the appended claims is hereby specifically incorporated in this specification.

### Brief description of the drawings

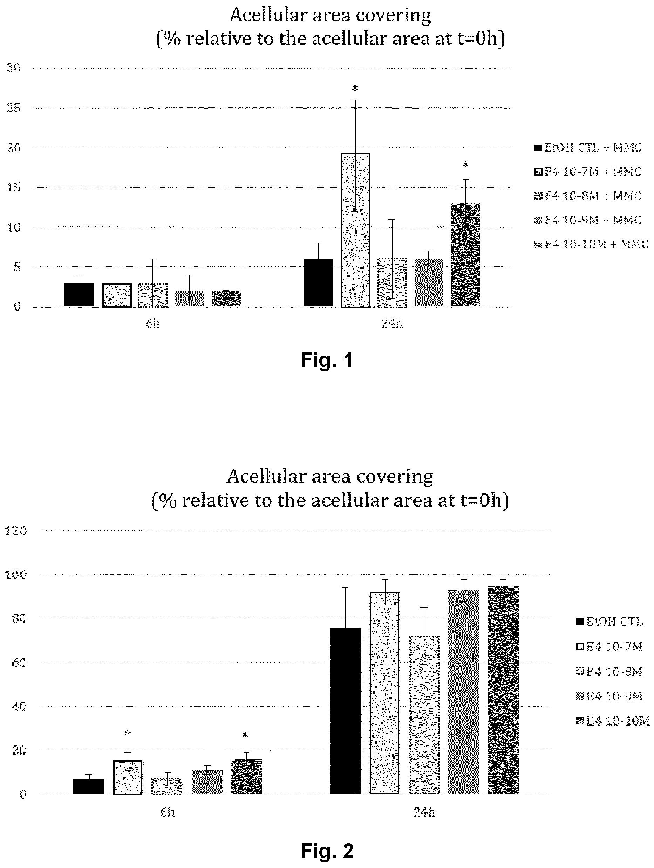

**Figure 1****.** Percentages of the acellular area covering by Normal Human Epidermal Keratinocytes (NHEKs) after different times of contact with estetrol (E4) applied at four concentrations, in presence of proliferation inhibitor mitomycin C (MMC). The results are expressed in percentage of the acellular area covering relative to time 0h (mean +/- standard deviations of three independent cultures). A t-student test was performed to compare the effects of the treatments to ethanol 0.1% + MMC. P values as 0.01 <p <0.05 are considered as significant, 0.001 <p <0.01 as highly significant and p <0.001 as very highly significant. Left condition: acellular area covering after 6 hours contact. Right condition: acellular area covering after 24 hours contact. For each time point, from left to right: EtOH control (CTL) + MMC; E4 10⁻⁷ M + MMC; E4 10⁻⁸ M + MMC; E4 10⁻⁹ M + MMC; E4 10⁻¹⁰ M + MMC.
**Figure 2****.** Percentages of the acellular area covering by Normal Human Epidermal Keratinocytes (NHEKs) after different times of contact with estetrol (E4) applied at four concentrations, in absence of proliferation inhibitor mitomycin C (MMC). The results are expressed in percentage of the acellular area covering relative to time 0h (mean +/- standard deviations of three independent cultures). A t-student test was performed to compare the effects of the treatments to ethanol 0.1%. P values as 0.01 <p <0.05 are considered as significant, 0.001 <p <0.01 as highly significant and p <0.001 as very highly significant. Left condition: acellular area covering after 6 hours contact. Right condition: acellular area covering after 24 hours contact. For each time point, from left to right: EtOH control (CTL); E4 10⁻⁷ M; E4 10⁻⁸ M; E4 10⁻⁹ M; E4 10⁻¹⁰ M.

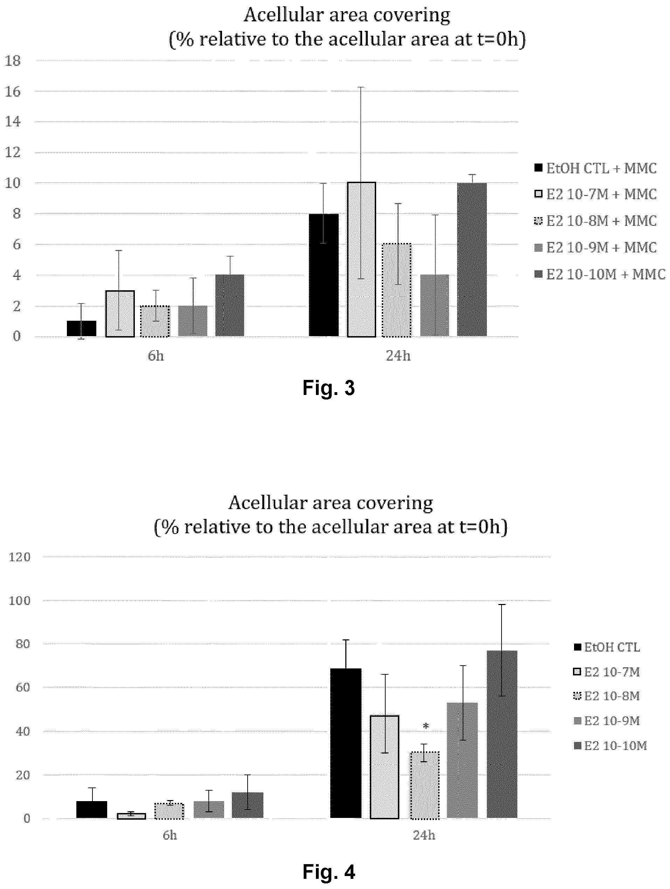

**Figure 3****.** Percentages of the acellular area covering by Normal Human Epidermal Keratinocytes (NHEKs) after different times of contact with beta-estradiol (E2) applied at four concentrations, in presence of proliferation inhibitor mitomycin C (MMC). The results are expressed in percentage of the acellular area covering relative to time 0h (mean +/- standard deviations of three independent cultures). A t-student test was performed to compare the effects of the treatments to ethanol 0.1% + MMC. P values as 0.01 <p <0.05 are considered as significant, 0.001 <p <0.01 as highly significant and p <0.001 as very highly significant. Left condition: acellular area covering after 6 hours contact. Right condition: acellular area covering after 24 hours contact. For each time point, from left to right: EtOH control (CTL) + MMC; E2 10⁻⁷ M + MMC; E2 10⁻⁸ M + MMC; E2 10⁻⁹ M + MMC; E2 10⁻¹⁰ M + MMC.
**Figure 4****.** Percentages of the acellular area covering by Normal Human Epidermal Keratinocytes (NHEKs) after different times of contact with beta-estradiol (E2) applied at four concentrations, in absence of proliferation inhibitor mitomycin C (MMC). The results are expressed in percentage of the acellular area covering relative to time 0h (mean +/- standard deviations of three independent cultures). A t-student test was performed to compare the effects of the treatments to ethanol 0.1%. P values as 0.01 <p <0.05 are considered as significant, 0.001 <p <0.01 as highly significant and p <0.001 as very highly significant. Left condition: acellular area covering after 6 hours contact. Right condition: acellular area covering after 24 hours contact. For each time point, from left to right: EtOH control (CTL); E2 10⁻⁷ M; E2 10⁻⁸ M; E2 10⁻⁹ M; E2 10⁻¹⁰ M.

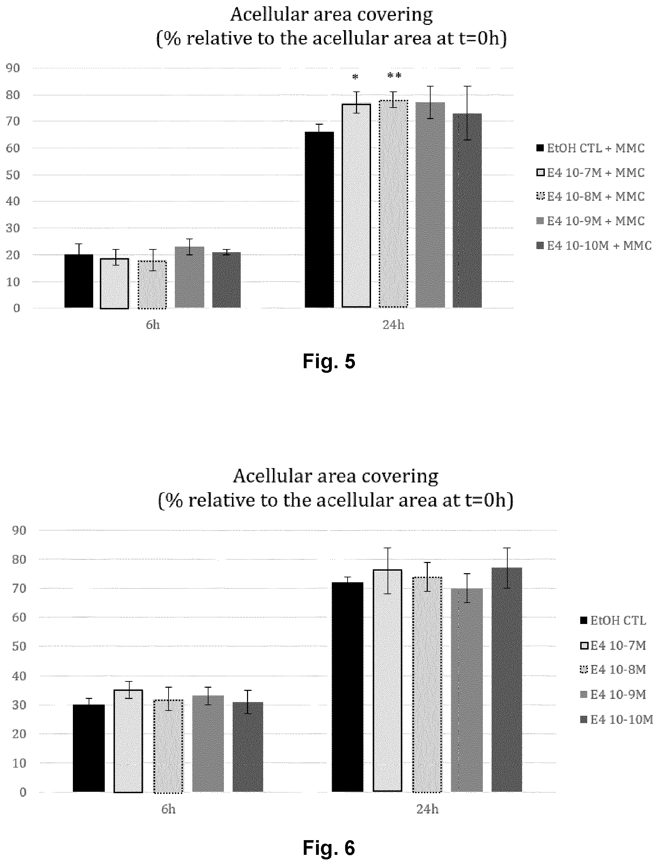

**Figure 5****.** Percentages of the acellular area covering by Normal Human Dermal Fibroblasts (NHDFs) after different times of contact with estetrol (E4) applied at four concentrations, in presence of proliferation inhibitor mitomycin C (MMC). The results are expressed in percentage of the acellular area covering relative to time 0h (mean +/- standard deviations of three independent cultures). A t-student test was performed to compare the effects of the treatments to ethanol 0.1% + MMC. P values as 0.01 <p <0.05 are considered as significant, 0.001 <p <0.01 as highly significant and p <0.001 as very highly significant. Left condition: acellular area covering after 6 hours contact. Right condition: acellular area covering after 24 hours contact. For each time point, from left to right: EtOH control (CTL) + MMC; E4 10⁻⁷ M + MMC; E4 10⁻⁸ M + MMC; E4 10⁻⁹ M + MMC; E4 10⁻¹⁰ M + MMC.
**Figure 6****.** Percentages of the acellular area covering by Normal Human Dermal Fibroblasts (NHDFs) after different times of contact with estetrol (E4) applied at four concentrations, in absence of proliferation inhibitor mitomycin C (MMC). The results are expressed in percentage of the acellular area covering relative to time 0h (mean +/- standard deviations of three independent cultures). A t-student test was performed to compare the effects of the treatments to ethanol 0.1%. P values as 0.01 <p <0.05 are considered as significant, 0.001 <p <0.01 as highly significant and p <0.001 as very highly significant. Left condition: acellular area covering after 6 hours contact. Right condition: acellular area covering after 24 hours contact. For each time point, from left to right: EtOH control (CTL); E4 10⁻⁷ M; E4 10⁻⁸ M; E4 10⁻⁹ M; E4 10⁻¹⁰ M.

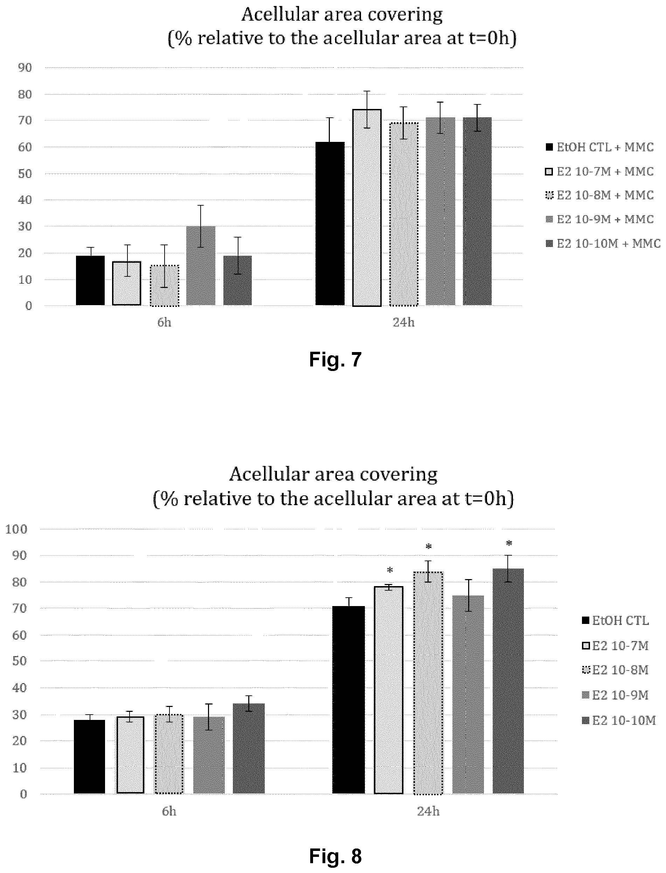

**Figure 7****.** Percentages of the acellular area covering by Normal Human Dermal Fibroblasts (NHDFs) after different times of contact with beta-estradiol (E2) applied at four concentrations, in presence of proliferation inhibitor mitomycin C (MMC). The results are expressed in percentage of the acellular area covering relative to time 0h (mean +/- standard deviations of three independent cultures). A t-student test was performed to compare the effects of the treatments to ethanol 0.1% + MMC. P values as 0.01 <p <0.05 are considered as significant, 0.001 <p <0.01 as highly significant and p <0.001 as very highly significant. Left condition: acellular area covering after 6 hours contact. Right condition: acellular area covering after 24 hours contact. For each time point, from left to right: EtOH control (CTL) + MMC; E2 10⁻⁷ M + MMC; E2 10⁻⁸ M + MMC; E2 10⁻⁹ M + MMC; E2 10⁻¹⁰ M + MMC.
**Figure 8****.** Percentages of the acellular area covering by Normal Human Dermal Fibroblasts (NHDFs) after different times of contact with beta-estradiol (E2) applied at four concentrations, in absence of proliferation inhibitor mitomycin C (MMC). The results are expressed in percentage of the acellular area covering relative to time 0h (mean +/- standard deviations of three independent cultures). A t-student test was performed to compare the effects of the treatments to ethanol 0.1%. P values as 0.01 <p <0.05 are considered as significant, 0.001 <p <0.01 as highly significant and p <0.001 as very highly significant. Left condition: acellular area covering after 6 hours contact. Right condition: acellular area covering after 24 hours contact. For each time point, from left to right: EtOH control (CTL); E2 10⁻⁷ M; E2 10⁻⁸ M; E2 10⁻⁹ M; E2 10⁻¹⁰ M.

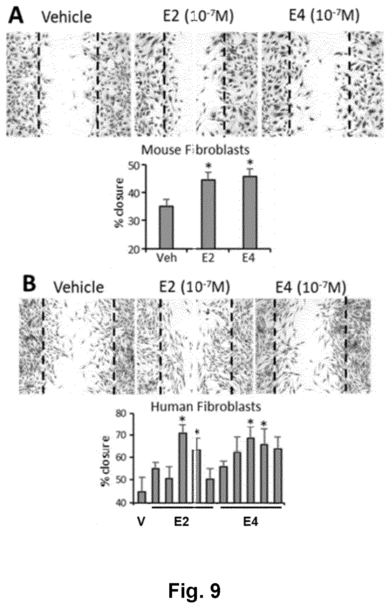

**Figure 9****.** Impact of estrogens on fibroblast migration in a scratch assay. Pilot experiments suggest that E4 is as effective as E2 in promoting fibroblast migration in both mouse (A) and human (B) fibroblasts. Top panels show representative images of wound closure in presence of vehicle, E2 (10⁻⁷ M) or E4 (10⁻⁷ M), bottom panels show the percentage of wound closure in presence of the vehicle, E2 or E4. E2 10⁻⁷ M and E4 10⁻⁷ M were tested in mouse fibroblasts (A), five doses were tested for both E2 and E4 in human fibroblasts (B), incrementally increasing from 10⁻¹⁰ M (left) to 10⁻⁶ M (right). Data are represented as mean +/- SEM of three independent cultures from different donors. *: p<0.05.

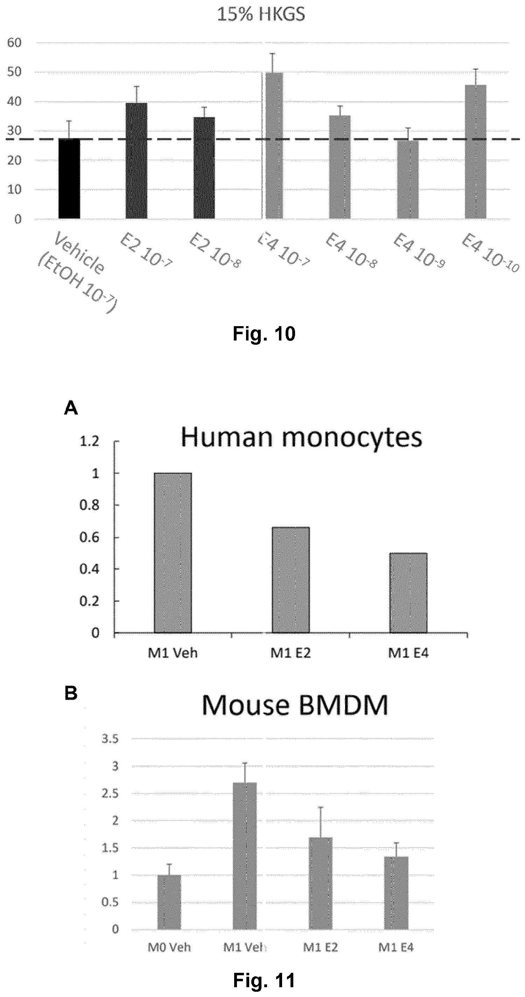

**Figure 10****.** Impact of estrogens on keratinocyte migration in a scratch assay. In a culture condition supplemented with 15% Human Keratinocyte Growth Supplement (HKGS) the impact of E4 (10⁻¹⁰ M and 10⁻⁷ M) on keratinocytes seems higher than the impact of E2. X-axis: different test conditions (all concentrations are expressed in M). Y-axis: percentage of wound closure. Dashed line: Basal keratinocyte migration level observed for control condition (outer left condition; 10⁻⁷ M ethanol).
**Figure 11****.** Impact of estrogens on immune cell polarization. Results are expressed as the relative expression of a pro-inflammatory marker representative of the subtype M1 in (A) activated human monocytes treated with a negative control (M1 Veh), estradiol (M1 E2) or estetrol (M1 E4), and in (B) non-activated mouse bone marrow derived monocytes (BMDM) treated with a negative control (M0 Veh) or activated mouse BMDM treated with a negative control (M1 Veh), estradiol (M1 E2) or estetrol (M1 E4).E4 administration results in a switch from monocyte subtype M1 (pro-inflammatory) to subtype M2 (healing).

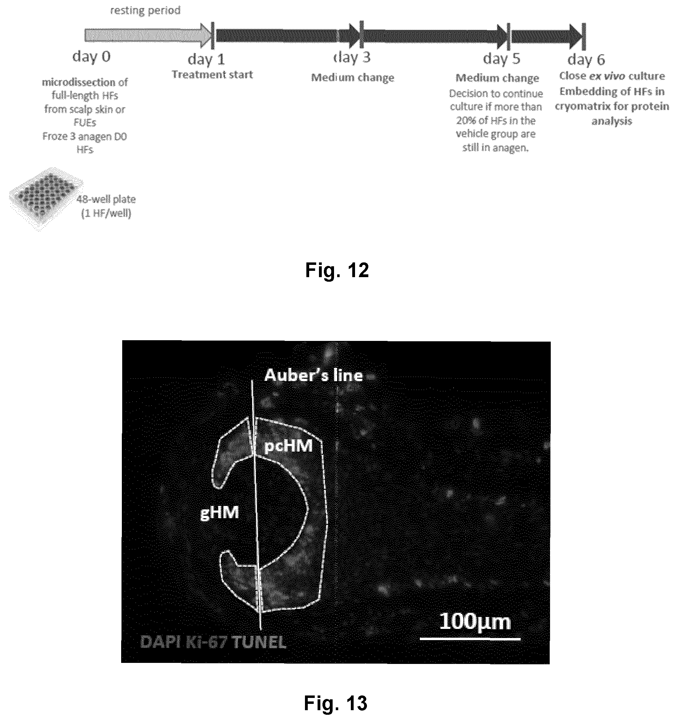

**Figure 12****.** Experimental design for Example 6. Microdissected human anagen VI HFs from FUEs were cultured at 37 °C with 5 CO₂ in a minimal media of William's E media (Gibco, Life Technologies) supplemented with 2 mM of L-glutamine (Gibco), 10 ng/ml hydrocortisone (Sigma Aldrich), 10 µ g/ml insulin (Sigma Aldrich) and 1 penicillin/streptomycin mix (Gibco) to make Williams Complete Media (WCM) as previously described (Edelkamp et al., Molecular Dermatology, 2020 and Langan et al., Exp Dermatol, 2015).
**Figure 13****.** Ki-67/TUNEL staining. gHM: germinative hair matrix; pcHM: precortical hair matrix.

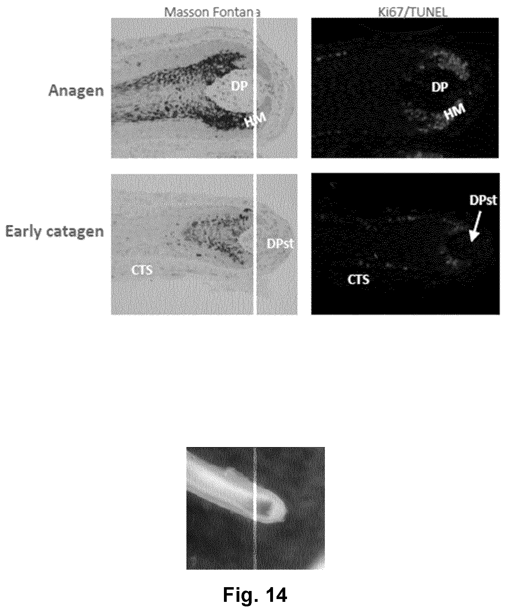

**Figure 14****.** Hair cycle stages. Top two images: anagen stage. Middle two images: early catagen stages. DP: dermal papilla; DPst: dermal papilla stalk; CTS: connective tissue sheath; gHM: germinative hair matrix. Bottom image: representative image of a dystrophic hair follicle (Keyence VHX900).

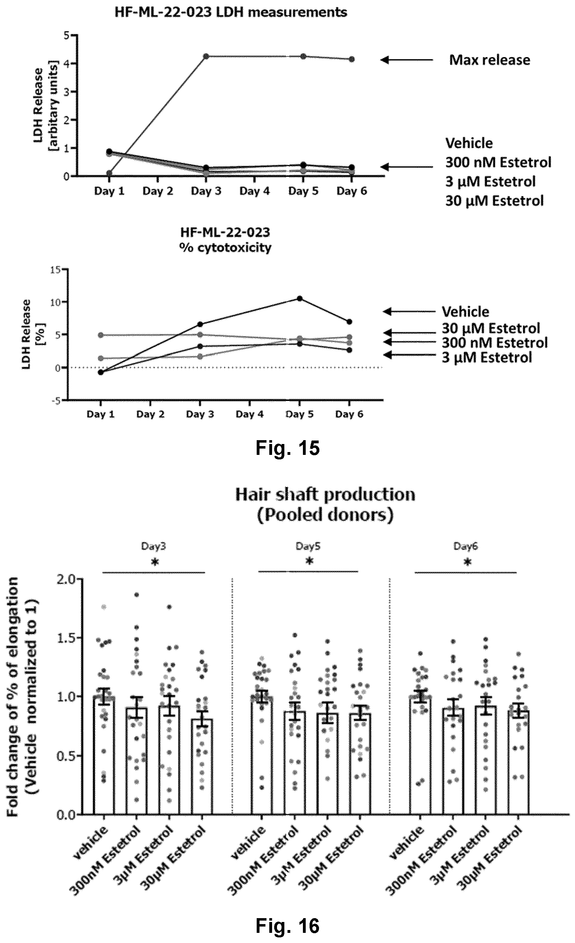

**Figure 15****.** Estetrol does not induce LDH release into the medium. Medium pooled from n= 7-8 HFs/group cultured until day 6 from Donor 2 (Day 1, Day 3, Day 5 and 6); Mean, GraphPad Prism 9, D'Agostino & Pearson omnibus normality test. A non-parametric analysis was applied, Kruskal Wallis test, Dunn's multiple comparison test - Vehicle fixed, not significant.

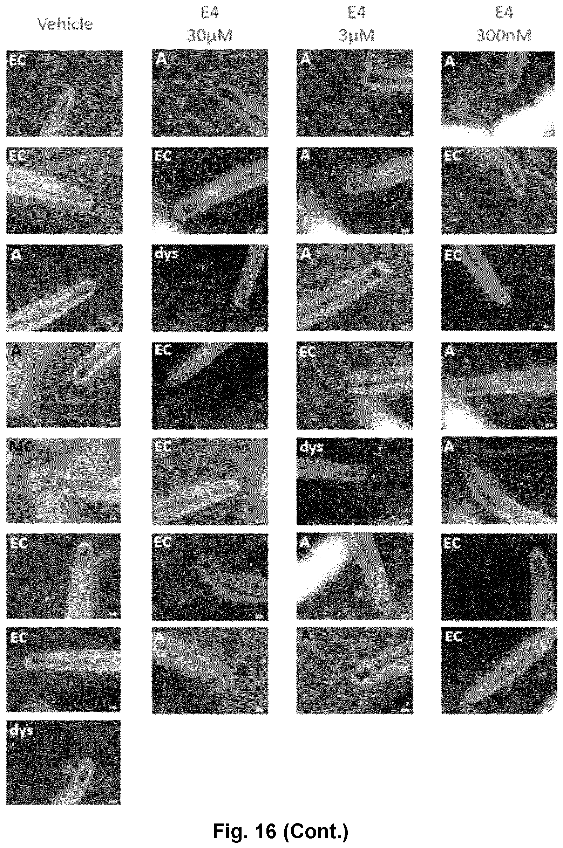

**Figure 16****.** Estetrol does not impact hair shaft production. Pool data of n= 4 female donors, Mean ± SEM, GraphPad Prism 9. Kruskal-Wallis test p=0.4856; Dunn's multiple comparison test (vehicle fixed) ns; Mann-Whitney vs vehicle *p<0.05. Macroscopic hair cycle staging was performed according to the criteria published in Langan et al., Exp Dermatol 2015. A = anagen; EC = early catagen; MC= mid catagen; dys= dystrophic catagen.

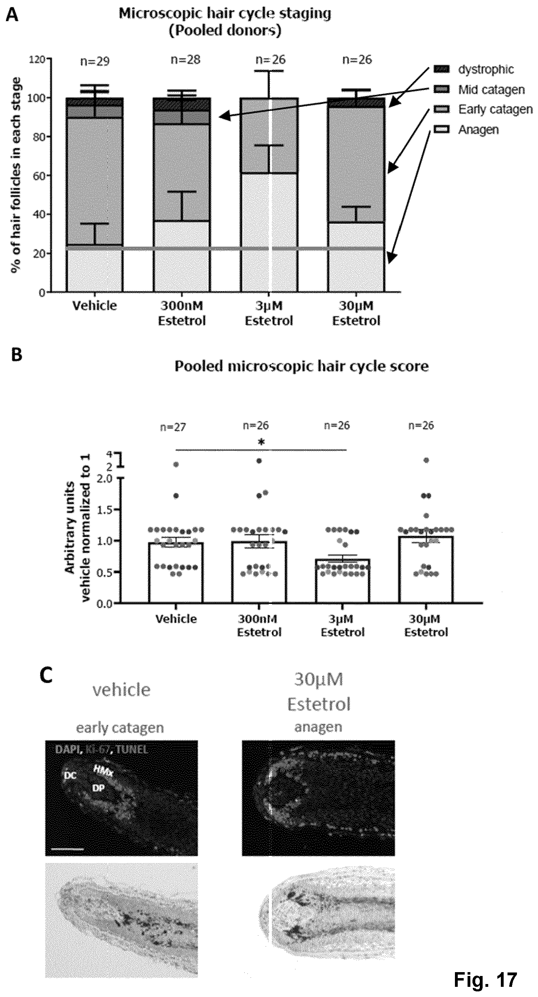

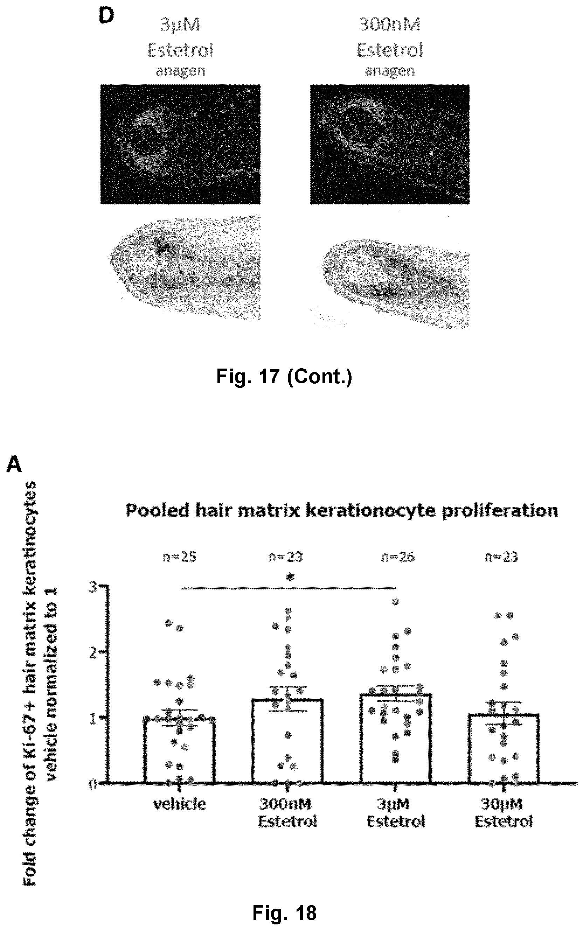

**Figure 17****.** Microscopically all estetrol concentrations (3 µM in particular) maintain longer HFs in anagen (A, B). Pool data of n= 4 female donors, Mean ± SEM, GraphPad Prism 9. Kruskal-Wallis test *p=0.0304; Dunn's multiple comparison test (vehicle fixed) ns; Mann-Whitney vs vehicle *p<0.05. (C, D) Microscopic images: DP: dermal papilla; HMx: hair matrix; DC: dermal cup. displayed scale bar indicates 100µm.

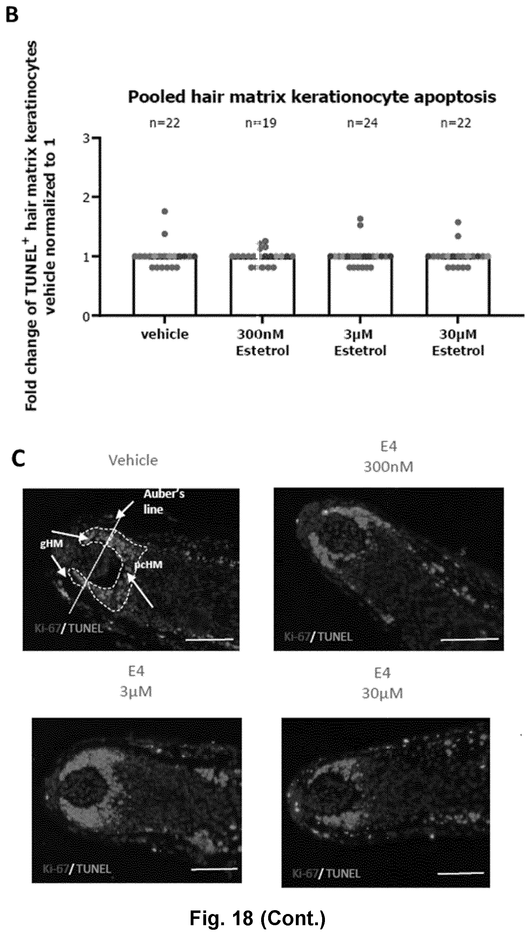

**Figure 18****.** Estetrol (3 µM) increases hair matrix keratinocytes proliferation, whilst apoptosis was not affected (A, B). Pool data of n= 4 female donors, Mean ± SEM, GraphPad Prism 9. (A): one-way ANOVA p=0.2115; Holm-Sida k's multiple comparison test vehicle fixed ns; Unpaired student's t-test vs vehicle *p<0.05. (B): Kruskal-Wallis test p=0.6460; Dunn's multiple comparison test (vehicle fixed) ns; Mann-Whitney vs vehicle; ns. (C) Microscopic images: Ki-67+ cells were counted in the demarcated areas below the Auber's line (germinative hair matrix, gHM) while TUNEL+ cells were counted in the demarcated areas below (gHM) and above (precortical hair matrix, pcHM) the Auber's line.

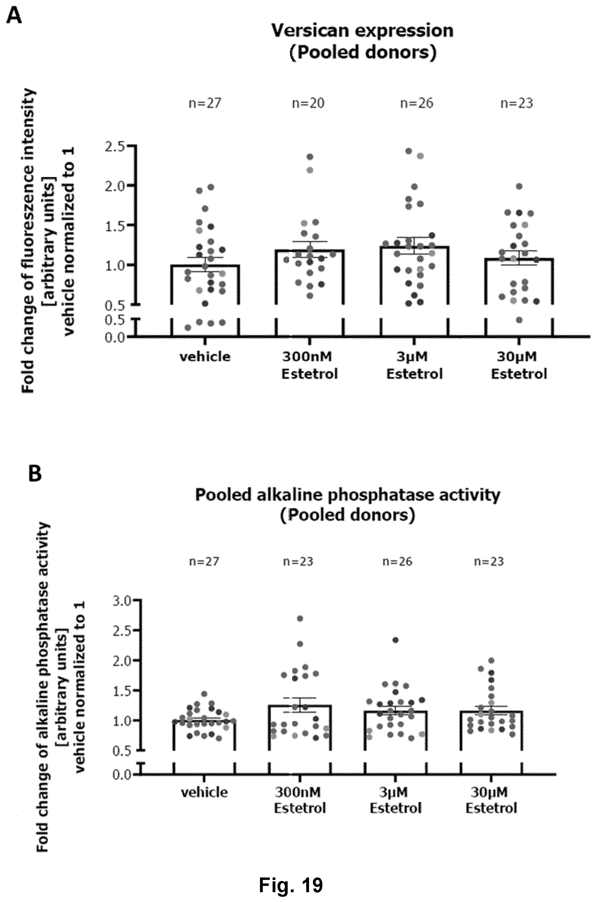

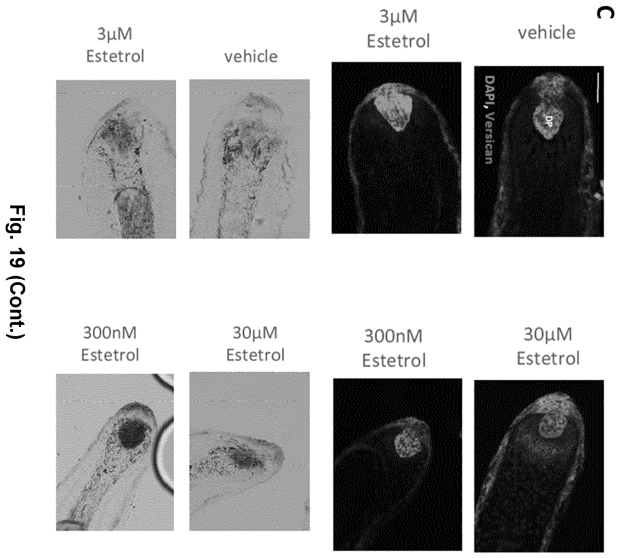

**Figure 19****.** All tested concentrations of E4 tendentially increased versican expression and alkaline phosphatase activity in dermal papilla (DP) cells. Pool data of n= 4 female donors, Mean ± SEM, GraphPad Prism 9 (A) versican expression in the dermal papilla. Kruskal-Wallis test p=0.3529; Dunn·s multiple comparison test (vehicle fixed) ns; Mann-Whitney vs vehicle ns. (B) Alkaline phosphatase activity in the dermal papilla. Kruskal-Wallis test p=0.5508; Dunn's multiple comparison test (vehicle fixed) ns; Mann-Whitney vs vehicle ns. (C) Microscopic images. DP= dermal papilla, displayed scale bar indicates 100µm.

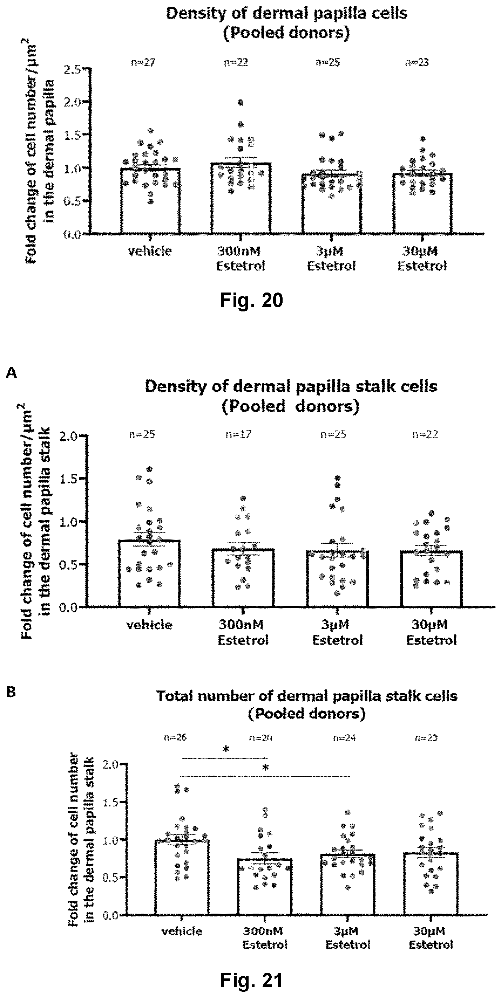

**Figure 20****.** DP fibroblast emigration analysis revealed that all tested concentrations of E4 did not strikingly affect the density of cells in the DP. Pool data of n= 4 female donors, Mean ± SEM, GraphPad Prism 9. Kruskal-Wallis test p=0.2081, Dunn's multiple comparison test (vehicle fixed) ns; Mann-Whitney vs vehicle ns.
**Figure 21****.** All Estetrol concentrations tendentially decreased the density of cells and 300nM and 3µM E4 significantly reduced the total number of cells in the DP stalk. Pool data of n= 4 female donors Mean ± SEM, GraphPad Prism 9. (A) Density of dermal papilla stalk cells. one-way ANOVA p=0.5092; Holm-Sidak's multiple comparison test vehicle fixed ns; Unpaired student's t-test vs vehicle ns. (B) Total number of dermal pailla stalk cells. one-way ANOVA *p=0.0296; Holm-Sida k's multiple comparison test vehicle fixed *p=0.0177; Unpaired student's t-test vs vehicle, *p<0.05.

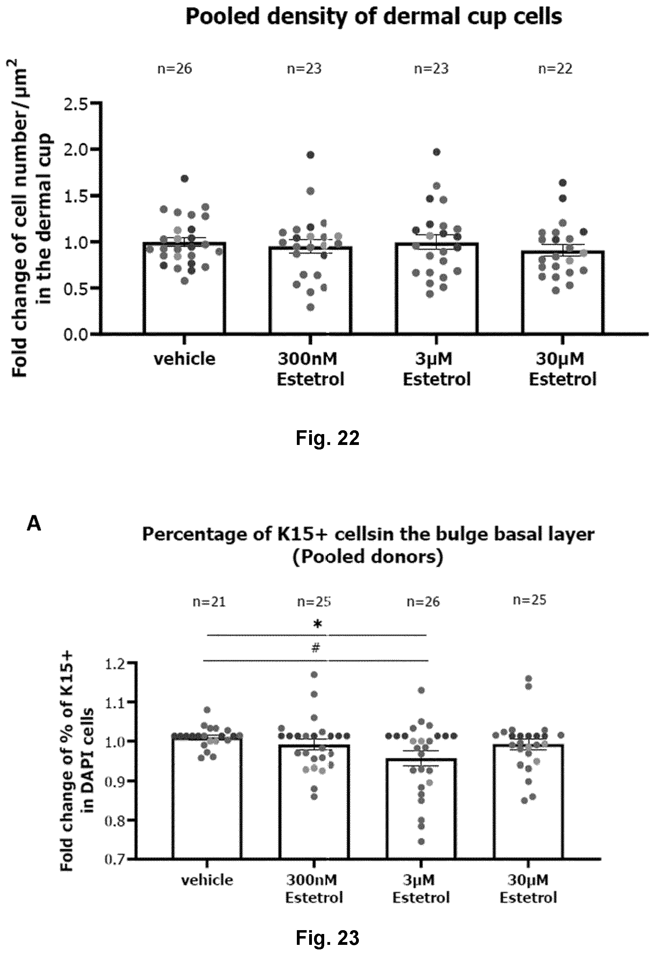

**Figure 22****.** Estetrol had no significant impact on the density of cells in the inductive dermal cup. Pool data of n= 4 female donors, Mean ± SEM, GraphPad Prism 9. One-way ANOVA p=0.7616; Holm-Sida k's multiple comparison test vehicle fixed ns; Unpaired student's t-test vs vehicle ns.

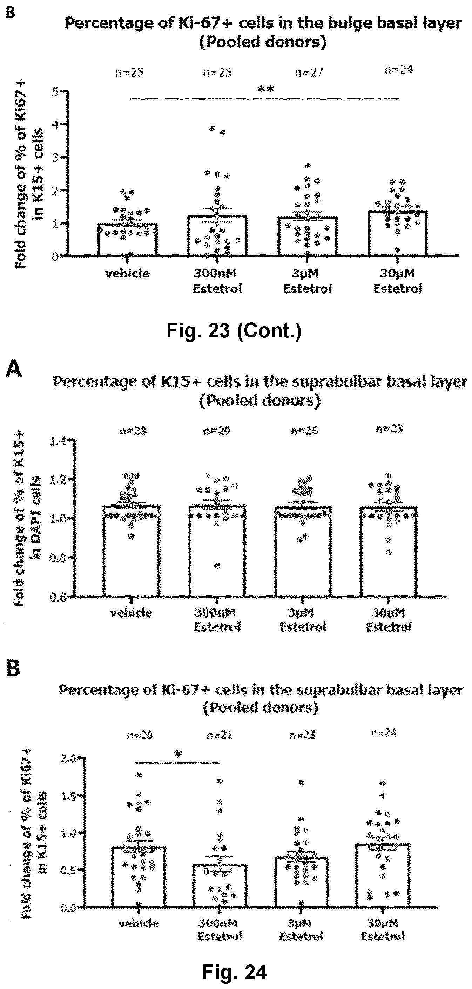

**Figure 23****.** In the bulge basal layer, E4 3µM significantly decreased the percentage of K15 positive cells, whilst E4 30µM (significantly) and 3µM and 300nM (tendentially) increased their proliferation. Pool data of n= 4 female donors, Mean ± SEM, GraphPad Prism 9. (A) Percentage of K15+ cells in the bulge basal layer. One-way ANOVA p=0.0641; Holm-Sidak's multiple comparison test vehicle fixed #p=0288; Unpaired student's t-test vs vehicle *p<0.05 (B) Percentage of Ki-67+ cells in the bulge basal layer. Kruskal-Wallis test p=0.1302; Dunn's multiple comparison test (vehicle fixed) ns; Mann-Whitney vs vehicle **p<0.01.

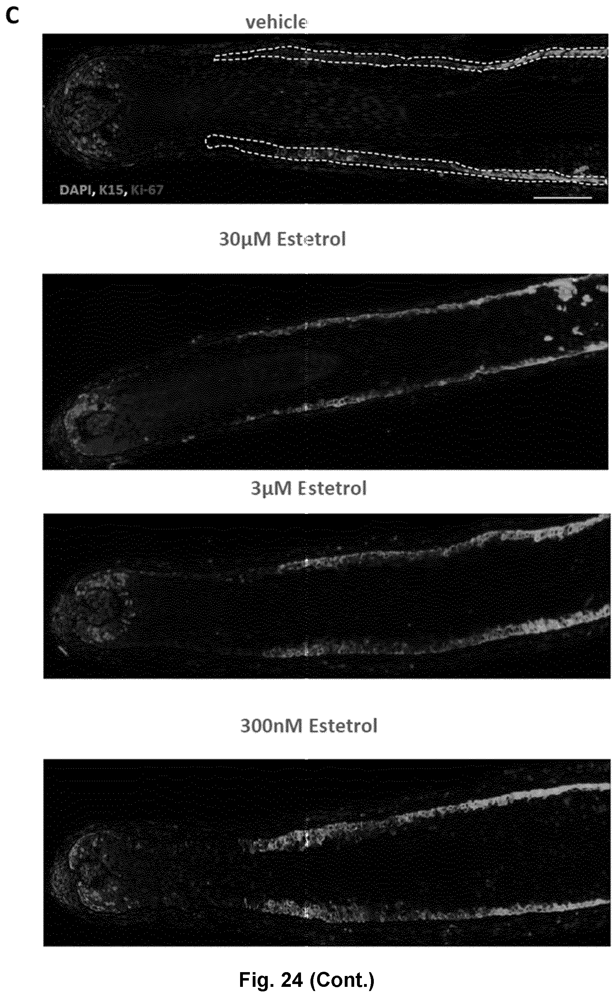

**Figure 24****.** In the suprabulbar ORS, estetrol did not have any effect on the percentage of K15 positive cells, but E4 300nM (significantly) and 3µM (tendentially) decreased their proliferation. Pool data of n= 4 female donors, Mean ± SEM, GraphPad Prism 9. (A) percentage of K15+ cells in the suprabulbar basal layer. Kruskal-Wallis test p=0.9375; Dunn's multiple comparison test (vehicle fixed) ns; Mann-Whitney vs vehicle ns. (B) Percentage of Ki-67+ cells in the suprabulbar basal layer. Kruskal-Wallis test p=0.7462; Dunn's multiple comparison test (vehicle fixed) ns; Mann-Whitney vs vehicle *p<0.05. (C) microscopic images. scale bar = 100µm (ORS: outer rooth sheath).

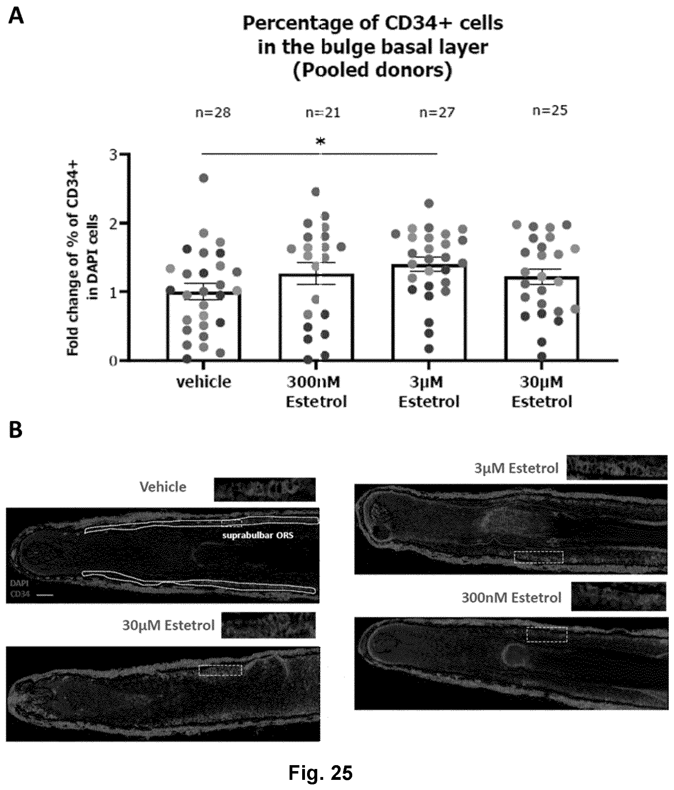

**Figure 25****.** Estetrol 3µM (significantly) and 300nM and 30µM (tendentially) increased the percentage of CD34 positive cells in the suprabulbar ORS. (A) percentage of CD34+ cells in the bulge basal layer. Pool data of n= 4 female donors, Mean ± SEM, GraphPad Prism 9 One-way A NOVA p=0.1011; Holm-Sida k's multiple comparison test vehicle fixed *p=0.0392; Unpaired student's t-test vs vehicle *p<0.05. (B) microscopic images. Scale bar = 100µm (ORS: outer root sheath).

### Detailed description of the disclosure

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. The terms also encompass "consisting of" and "consisting essentially of', which enjoy well-established meanings in patent terminology.

The recitation of numerical ranges by endpoints includes all numbers and fractions subsumed within the respective ranges, as well as the recited endpoints. This applies to numerical ranges irrespective of whether they are introduced by the expression "from... to..." or the expression "between... and..." or another expression.

The terms "about" or "approximately" as used herein when referring to a measurable value such as a parameter, an amount, a temporal duration, and the like, are meant to encompass variations of and from the specified value, such as variations of +/-10% or less, preferably +/-5% or less, more preferably +/-1% or less, and still more preferably +/-0.1% or less of and from the specified value, insofar such variations are appropriate to perform in the disclosure. It is to be understood that the value to which the modifier "about" or "approximately" refers is itself also specifically, and preferably, disclosed.

Whereas the terms "one or more" or "at least one", such as one or more members or at least one member of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members. In another example, "one or more" or "at least one" may refer to 1, 2, 3, 4, 5, 6, 7 or more.

The discussion of the background to the disclosure herein is included to explain the context of the disclosure. This is not to be taken as an admission that any of the material referred to was published, known, or part of the common general knowledge in any country as of the priority date of any of the claims.

Unless otherwise defined, all terms used in the present disclosure, including technical and scientific terms, have the meaning as commonly understood by one of ordinary skill in the art to which this disclosure belongs. By means of further guidance, term definitions are included to better appreciate the teaching of the disclosure. When specific terms are defined in connection with a particular aspect of the disclosure or a particular example of the disclosure, such connotation or meaning is meant to apply throughout this specification, i.e., also in the context of other aspects or examples of the disclosure, unless otherwise defined. For example, examples directed to products are also applicable to corresponding features of methods and uses.

In the following passages, different aspects or examples of the disclosure are defined in more detail. Each aspect or example so defined may be combined with any other aspect(s) or example(s) unless clearly indicated to the contrary. In particular, any feature indicated as being preferred or advantageous may be combined with any other feature or features indicated as being preferred or advantageous.

Reference throughout this specification to "one example", "an example" means that a particular feature, structure or characteristic described in connection with the example is included in at least one example of the present disclosure. Thus, appearances of the phrases "in one example" or "in an example" in various places throughout this specification are not necessarily all referring to the same example. Furthermore, the particular features, structures or characteristics may be combined in any suitable manner, as would be apparent to a person skilled in the art from this disclosure, in one or more examples. Furthermore, while some examples described herein include some but not other features included in other examples, combinations of features of different examples are meant to be within the scope of the disclosure, and form different examples, as would be understood by those in the art. For example, in the appended claims, alternative combinations of claimed examples are encompassed, as would be understood by those in the art.

### General definitions and context of the disclosure

The term "estetrol component", as used throughout this document, encompasses substances selected from the group consisting of estetrol, esters of estetrol, esters of estetrol wherein the hydrogen atom of at least one of the hydroxyl groups has been substituted by an acyl radical of a hydrocarbon carboxylic, sulfonic acid or sulfamic acid of 1-25 carbon atoms, estetrol hydrates such as estetrol monohydrate; and combinations thereof. It is understood that when estetrol is mentioned throughout any section of this specification, any estetrol-containing component (i.e. compound) and/or estetrol derivative (such as an estetrol ester) is also envisaged. More preferably, in the context of the present disclosure, a particularly preferred estetrol component suitable for the dosage unit or the cosmetic or medical uses and methods of treatment described herein is estetrol (including estetrol hydrates). Most preferably, said estetrol component is estetrol monohydrate.

The term "estetrol" as used herein refers to 1,3,5 (10)-estratrien-3,15alpha,16alpha,17beta-tetrol or 15alpha-hydroxyestriol as well as hydrates of estetrol, e.g. estetrol monohydrate. "Estetrol", or short "E4" is an estrogen steroid produced by the foetal human liver (PubChem CID: 27125). Estetrol may be described as a 3-hydroxy steroid corresponding to 17beta-estradiol wherein the 15α and 16α positions are substituted for two additional hydroxy groups. It is known that estetrol is an estrogen receptor agonist (Coelingh Bennink et al., Estetrol review: profile and potential clinical applications, Climacteric, 2008). In instances wherein the estetrol component described herein indicates estetrol, said estetrol may be endogenous estetrol. Alternatively, the estetrol may be chemically synthesized, synthesized by the use of (mutant) recombinant enzymes, or synthesized by any combination thereof. Estetrol may alternatively be indicated in the art by its molecular formula: C₁₈H₂₄O₄, or by structural formula (I):

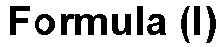

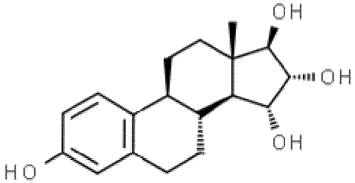

In a preferred example, the estetrol is present or used herein as a monohydrate.

In some examples, the estetrol component can be the sole active ingredient or can be combined with any other cosmetic or pharmaceutically active agent for alleviating or preventing hair loss known in the art.

Although in a preferred example, said estetrol component is administered without a progestogenic component, in some examples, e.g. when the patient still has a uterus, an optional progestogenic component may be administered in addition to the estetrol component.

The terms "progestogen", "progestogen", "gestagen", or "gestogen" and derived hereof "progestogenic compounds" as used both herein and in the art refer to any molecule that produces effects similar to those of the natural female sex hormone progesterone in the body of a subject. Progestogens are considered to be agonists of the progesterone receptors and their functions have been thoroughly examined in the art (inter alia discussed in Kuhl, Pharmacology of estrogens and progestogens: influence of different routes of administration, Climacteric, 2005). Progestins are a subgroup of progestogens that comprise synthetic progestogens. While the above terms may be used interchangeably in the art, there is a general understanding that when progestin is mentioned, synthetic progestogens are meant.

Examples of progestins are: levonorgestrel, norgestimate, norethisterone, dydrogesterone, drospirenone, 3-beta-hydroxydesogestrel, 3-ketodesogestrel, 17-deacetylnorgestimate, 19-norprogesterone, acetoxypregnenolone, allylestrenol, amgestone, chlormadinone, cyproterone, demegestone, desogestrel, dienogest, dihydrogesterone, dimethisterone, ethisterone, ethynodiol diacetate, fluorogestone acetate, gastrinone, gestodene, gestrinone, hydroxymethylprogesterone, hydroxyprogesterone, lynestrenol, mecirogestone, medroxyprogesterone, megestrol, melengestrol, nomegestrol, norethindrone, norethynodrel, norgestrel (including d-norgestrel, and dl-norgestrel), norgestrienone, normethisterone, progesterone, quingestanol, (17 alpha)-17-hydroxy-11-methylene-19-norpregna-4, 15-dien-20-yn-3-one, tibolone, trimegestone, algestone-acetophenide, nestorone, promegestone, 17-hydroxyprogesterone esters, 19-nor-17hydroxyprogesterone, 17alpha-ethynyltestosterone, 17alpha-ethynil-19-nortestosterone, d-17beta-acetoxy-13beta-ethyl-17alpha-ethynylgon-4-en-3-one oxime, 6beta, 7beta;15beta,16beta-dimethylene-3-oxo-17-pregna-4,9(11)-diene-21, 17beta-carbolactone or tanaproget and precursors of these compounds that are capable of liberating these progestogens in vivo.

Drospirenone (abbreviated as DRSP, PubChem CID: 68873) is an example of a progestin and enjoys a widespread use in Combined Oral Contraceptices COCs due to its antimineralocorticoid and antiandrogenic activity combined with a general low off-target activity. In general, drospirenone-containing COCs are referred to as fourth generation COCs. Non-limiting examples of commercially available COCs comprising drospirenone are known as "Yaz^{™}" and Yasmin^{™}. An illustrative example of a drospirenone only progestogen pill is "Slynd^{™}", which is also commercially available. Additionally, HRT compositions comprising an estrogen such as estradiol and drospirenone are available such as "Angeliq^{™}". Drospirenone may alternatively be indicated in the art by its molecular formula C₂₄H₃₀O₃, or by the structural formula (II):

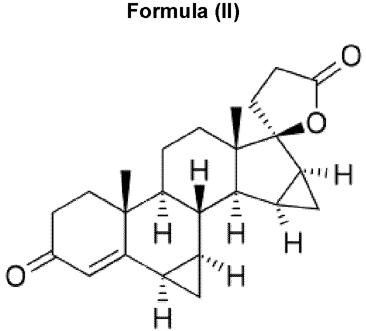

It is understood that when the term "drospirenone" is used herein, any drospirenone derivatives are also envisaged.

By means of illustration and not limitation, other progestogens or progestins that have been used in COCs include norethisterone, norethindrone, levenogestrel (LNG), norgestrel, gestodene, desogestrel, norgestimate, cyproterone acetate, dienogest, and chlormadinone.

In the context of the present disclosure, other compounds may be used in conjunction with the estetrol component for administering to women who have an uterus. Selective Estrogen Receptor Modulators (SERMs) defines a category of such compounds, which are contemplated as useful complements to the estetrol component in the methods of the disclosure. A preferred SERM for use in the context of the present disclosure is bazedoxifene.

In the methods and compositions further described herein, it has to be understood that when reference is made to a "progestogenic component", such reference includes SERMs and in particular bazedoxifene.

The term "an effective amount" refers to an amount necessary to obtain a physiological effect. The physiological effect may be achieved by one dose or by repeated doses.

Preferred subjects are female human subjects.

In certain examples, the subject is a female menopausal, perimenopausal, and/or postmenopausal subject. In certain examples, the subject is a menopausal, perimenopausal, or postmenopausal subject having an estradiol level of less than 100 pg/ml, preferably less than 50 pg/ml, preferably less than 30 pg/ml, more preferably less than 20 pg/ml, more preferably less than 20 pg/ml, most preferably less than 10 pg/ml. In alternative examples, the subject is a menopausal, perimenopausal, or postmenopausal subject that is characterised by having follicle-stimulating hormone concentrations of at least 20 milli-international units per millilitre (mIU/ml), preferably at least 25 mlU/ml, more preferably at least 30 mlU/ml, more preferably at least 35 mIU/ml, most preferably at least 40 mlU/ml.

"Menopausal subjects", used interchangeably in the art with "post-menopausal subjects" or "climacteric subjects" are female subjects that that have not had menstrual bleeding for a year which is accompanied by a decrease or discontinuation in hormone production by the ovaries (such as estradiol). Alternatively worded, "menopause" may be described as a biological condition characterised by impairment or cessation of ovarian primary function. Menopause may be accompanied by a broad range of clinical symptoms which are variable in severity such as but not limited to vasomotor dysfunction, vaginal dryness, mood changes, sleep disturbances, urinary incontinence, cognitive changes, somatic complaints, and sexual dysfunction. Methodologies to diagnose menopause have been described in the art and are therefore known to a person skilled in the art (Nelson, Menopause, Lancet, 2008).

"Perimenopause" refers to a period of life which begins approximately three to four years prior to menopause and ends one year after the final menstrual period, and is characterised by persistent irregular menstrual cycles, extreme fluctuations in hormonal levels, frequent anovulation and the appearance of vasomotor symptoms (Harlow et al., Executive summary of the Stages of Reproductive Aging Workshop + 10: addressing the unfinished agenda of staging reproductive aging, Menopause, 2012). The term "post menopause" or "postmenopausal" is indicative for female subjects that are characterised by a permanent cessation of menstrual periods. This permanent cessation is determined retrospectively after an observation of 12 months of amenorrhea without any other obvious pathological or physiological cause. The term "post menopause" also includes menopause as the consequence of premature ovarian failure, surgery (ovariectomy for example), chemotherapy or radiotherapy for cancer, and certain diseases (for example, infections or hypothyroidism).

The term "hair loss" as used herein corresponds to hair loss resulting from reduction in estrogen levels in a subject, typically following the menopausal transition. The term corresponds to female pattern hair loss and female androgenic alopecia in general. It can be occurring due to natural menopausal events or due to early removal of the uterus or any other syndrome or disorder leading to hypoestrogenic state. Although obviously the above only applies to female subjects, alopecia can also occur in male subjects where estrogen levels primarily resulting from testosterone conversion are below normal levels.

The present inventors have unexpectedly found that estetrol has a positive effect on the proliferation and functioning of keratinocytes.

The term "keratinocytes" encompasses a type of cells found in the epidermis and in the epithelial hair follicle cells encompassing bulge keratinocytes, outer root sheath (ORS) keratinocytes and hair bulb keratinocytes. In the outer layer of the skin, keratinocytes constitute about 90% of cells. The term "increasing keratinocyte function" is meant to include both increased proliferation of keratinocytes in the epidermis and hair follicles as well as increasing, improving or restoring the function of keratinocytes in the epidermis and hair follicles. Said function includes, but is not limited to, skin thickness and hydration, providing an adequate skin barrier and ensuring proper wound healing, promoting growth of hair follicle cells during anagen and epithelial-mesenchymal interaction, i.e. interaction with mesenchymal cells such as fibroblasts of the hair follicle, including dermal papilla cells.

Keratinocytes also play a role in oxidation and inflammation. Elevation in ROS production (generated amongst others in keratinocytes) and oxidative stress increase damage to DNA, proteins and lipids, and can lead to premature skin aging and hair loss. Chronic low-grade inflammation damages the skin by increasing the expression of proinflammatory cytokines by keratinocytes leading to detrimental changes and premature skin aging and hair loss.

Furthermore, skin pigmentation is influenced by keratinocytes as well as melanocytes, whose primary function is the production of the melanin pigment, are surrounded by keratinocytes, to which they transfer their melanin pigment. Due to the tight interaction and communication between melanocytes and keratinocytes, a single melanocyte can be surrounded by 36 keratinocytes, influencing keratinocytes e.g. by estetrol also influences melanocytes and hence pigmentation. The term "melanin" as used in the present disclosure refers to a group of natural pigments produced by melanocytes that are localized in the epidermis. Different types of melanin have been described in the art (e.g. in Wei et al., Unraveling the structure and function of melanin through synthesis, J Am Chem Soc, 2021). "Melanogenesis" is commonly used in the art and relates to the process of melanin production. Melanogenesis is for example upregulated upon exposure of the skin to UV radiation and results in a darkening of the skin tone.

The term "reactive oxygen species", commonly abbreviated as "ROS" is an umbrella term for molecules formed from oxygen (O₂). Illustrative examples include peroxides, superoxides, hydroxyl radicals, singlet oxygens, and alpha oxygens. Reactive Oxygen Species are, among others, produced in fibroblasts and keratinocytes. Both the dermis and epidermis can protect the skin against oxidation, while the epidermis contains the highest concentration of antioxidants. ROS is a negative or aggravating factor in terms of female pattern hair loss due to damage caused to the hair follicles. Reducing ROS is hence important for avoiding female pattern hair loss.

Keratinocytes are also an important cell type for promoting wound healing, as they are responsible for the wound closure and the re-epithelialization but also for modulating inflammation in the wound next to immune cells. An imbalance in keratinocyte function can lead to imbalanced inflammation.

Expressions relating to "inflammation of the skin" or "skin inflammation" used herein are to be interpreted according to the commonly accepted meaning in the state of the art and thus indicate any local immune response of the skin. The cause of the skin inflammation is not particularly limited in the context of the present disclosure and include e.g. pathogens, immune system dysfunction, allergic reactions, and wounds. Skin inflammation can therefore be considered the result of cellular interactions in the skin of a subject, with immune cells remaining the most important cell type.

Additionally, the inventors have found that estetrol also positively influences the proliferation and functioning of fibroblasts.

The term "fibroblasts" encompasses a type of cells found in the dermis and in the mesenchymal hair follicle cells encompassing dermal papilla cells. Such fibroblast also include dermal papilla cells in the hair follicle which, although virtually not proliferating, are growing in number through interactions with surrounding keratinocytes due to epithelial mesenchymal interactions (EMI).

Dermal fibroblasts are the main cell type present in skin connective tissue (dermis). Fibroblasts interact with epithelial cells during hair development (EMI) and in interfollicular skin, play an essential role during cutaneous wound healing, and are known to have different functions in maintaining good health of the skin, more particularly in maintaining skin thickness, skin collagen content and elasticity, and maintaining a good balance of skin water content/loss. Fibroblasts interact with epidermal cells during hair development and in interfollicular skin.

"Sebum" as used herein refers to an oily and/or waxy substance which is produced in the sebaceous glands by sebocytes, the latter being highly specialized epithelial cells that secrete sebum by means of holocrine secretion. Sebum is a composition comprising triglycerides, wax esters, squalene, and free fatty acids, and forms an integral component of the epidermal barrier and immune system of the skin. Sebaceous glands develop from the same tissue that gives rise to the epidermis and can be stratified into sebaceous glands connected to hair follicles and independently existing sebaceous glands. The secretions of the sebaceous glands (sebum) provides emollients for the hair and skin and are under the influence of androgen hormones. Sebum secretion declines with age and can be influenced by estrogen levels.

### Anti-hair loss therapy and improvement in hair texture, hair quality, and hair appearance

The present disclosure provides in an aspect a composition comprising an estetrol component for use in preventing or treating hair loss as defined herein elsewhere. Alternatively worded, the present disclosure envisages the use of a composition comprising an estetrol component for preventing or treating hair loss, and improvement in hair texture, quality and appearance. Yet alternatively worded, the present disclosure envisages use of an estetrol component (or a composition comprising an estetrol component) for the manufacture of a medicament for preventing or treating hair loss. Finally, the disclosure further provides a method of treatment for preventing or reducing hair loss comprising administration of a composition comprising systemic administration of an estetrol component to a subject. Numerous methods and approaches to measure and/or monitor hair loss have been described in the art (reviewed by e.g. Dhurat and Saraogi, Int J Trichology, 2009), such as target area hair count ("TAHC") and target area hair width ("TAHW"). The terms target area hair width and target area hair thickness may be used interchangeably herein and throughout the art.

The term "hair loss" as used herein relates to any kind of hair loss in a subject, preferably an adult subject, more preferably a female adult subject. "Hair loss" as used herein broadly refers to any kind of alopecia. Thus, "hair loss" as used herein encompasses the clinical conditions better known as androgenetic alopecia (AGA), or alopecia areata, fibrosing alopecia, diffuse alopecia, scarring alopecia, and universal alopecia. Hence, the dosage unit for use, the use, of the method described herein may be used for treating alopecia, in particular androgenetic alopecia. Androgenetic alopecia has been described in great detail in the art at numerous occasions and is therefore known to a person skilled in the art (for example in Chin et al., Androgenetic alopecia, StatPearls, 2022). Androgenetic alopecia (interchangeably indicated by the term "pattern hair loss") is a hair loss condition that mainly affects the top and front of the scalp. Male-pattern hair loss is generally described as a receding front hair line, hair loss on the crown of the scalp, or a combination thereof. Female-pattern hair loss is typically characterised by a diffuse thinning across the scalp. In preferred examples, the dosage unit for use, the use, or the method described herein is used for treating female-pattern hair loss.

The terms "treatment" or "treat" are to be interpreted as both the therapeutic treatment of a symptom, disease or condition that has already developed, leading to (clinical) manifestations, as well as prophylactic or preventive measures, wherein the goal of the treatment is to prevent, lessen, or reduce the chances of incidence of an undesired affliction, such as to prevent occurrence, development and progression of symptoms, (clinical) conditions or diseases related to hair loss. Therefore, a further aspect of the disclosure is directed to an effective amount of an estetrol component for use in the prevention of hair loss in a subject, preferably a menopausal subject. Beneficial or desired clinical results may include, without limitation, alleviation of one or more symptoms, improvement of one or more biological markers, diminishment of extent of disease, stabilized (i.e. not worsening) state of disease, delay or slowing of disease progression, amelioration or palliation of the disease state, and the like.

"Prevention" or "prevent" as used in the context of the disclosure refers to an aversion of manifestation of a condition or disease image in a subject, i.e. the establishment of preventive measures or prophylactic measures. Preventive treatment refers to treatments wherein the object is to avoid a subject's body or an element thereof to show (worsening of) symptoms of an undesired physiological change.

As used herein, the terms "therapeutic treatment" or "therapy" and the like, refer to treatments wherein the aim is to change a subjects body or a part of a subjects body from an undesired physiological state, disease or disorder which is caused by aging, to a desired state, such as a less severe state (e.g., amelioration, or even back to its normal, healthy state (e.g., restoring the health, the physical integrity and the physical well-being of a subject), to keep it (i.e., not worsening) at said undesired physiological status (e.g., stabilization), or slow down progression to a more severe or worse state compared to said undesired physiological change or disorder. Measurable lessening includes any statistically significant decline in a measurable marker or symptom. Statistically significant as used herein refers to p values below 0.05, which is a commonly accepted cut-off score in statistical analysis as a skilled person appreciates. "Treatment" encompasses both curative treatments and treatments directed to reduce symptoms and/or slow progression and/or stabilize the condition or disease.

A skilled person is aware that in order to achieve an effective therapeutic treatment, a therapeutically effective dose needs to be administered to said subject. Therefore, in the context of the present disclosure "an effective amount" refers to an amount necessary to obtain a physiological effect. The physiological effect may be achieved by a single dose or by multiple doses. A "therapeutically effective amount" or "therapeutically effective dose" indicates an amount of estetrol component that when administered brings about a clinical positive response with respect to treatment of a subject afflicted by hair loss. Similarly, a "prophylactically effective amount" or "prophylactically effective dose" refers to an amount of estetrol component that inhibits or delays the onset of (clinical) manifestation of hair loss. A skilled person is aware that terms such as "quantity", "amount" and "level" are synonyms and have a well-defined meaning in the art and appreciates that these may particularly refer to an absolute quantification of an estetrol component which is considered an effective amount for the applications described herein, or to a relative quantification of the estetrol component, such as for example a concentration of estetrol component in function of the subject's bodyweight. Suitable values or ranges of values may be obtained from one single subject or from a group of subjects (i.e. at least two subjects).

It is emphasised that while any of the herein disclosed values and ranges of the estetrol components are suitable for the different medical indications or purposes, a skilled person is aware that certain individuals may experience yet improved benefits from the estetrol component treatment by further optimisation optimal dose of said component by considering a wide range of parameters including but by no means limited to the nature and degree of the condition or disease to be treated, gender of the subject, subject age, body weight, other medical indications, nutrition, mode of administration, metabolic state, interference or influence by or efficacy of other pharmaceutically active ingredients, etc. Furthermore, each individual may have a certain intrinsic degree of responsiveness to the estetrol component that is used.

Terms such as "subject", "patient", "individual" may be used interchangeably herein and refer to human subjects, preferably female subjects that are in the menopausal or perimenopausal phase of their life.

Equally envisaged are cosmetic methods to prevent or reduce hair loss comprising a step of systemic administration of a composition comprising an estetrol component. Alternatively worded, the disclosure equally envisages cosmetic use of a composition comprising an estetrol component for preventing or reducing hair loss. A skilled person appreciates that "cosmetic use" implies a "non-medical, non-therapeutic use", thus a use that does not aim to ameliorate, prevent or treat a clinical pathology.

The present therapeutic or cosmetic therapy usually employs continuous administration of the estetrol component during a period of at least 10 days, preferably of at least 20 days.

The estetrol therapeutic or cosmetic component is administered at a daily dose of from about 10 mg to about 25 mg of the estetrol component, preferably estetrol, more preferably estetrol monohydrate.

In a specific example, the estetrol component is administered at a daily dose of from about 14 mg to about 21 mg.

In another specific example, the estetrol component is administered at a daily dose of from about 14 mg to about 16 mg or from about 19 mg to about 21 mg.

In one example, the present therapeutic or cosmetic therapy is administered to non-hysterectomized patients. In a particular example, the present therapeutic or cosmetic therapy involves daily administration of about 15 mg or 20 mg of the estetrol component, preferably to non-hysterectomized patients.

In the cases when the present therapeutic or cosmetic therapy is administered to a patient who has undergone hysterectomy, the estetrol component is preferably administered without a progestogenic component or even as the sole active ingredient.

When the present therapy is administered to non-hysterectomized patients, the estetrol component may be administered as sole active ingredient or may be administered together with an optional progestogenic component. Said optional progestogenic component may be administered continuously (i.e. every day in addition to the estetrol component) or sequentially (wherein sequentially means an administration of the progestogenic component during, for example, 10 to 14 days each month or during 14 days every 3 months).

The terms "continuous" / "continuously" as used herein, means that the components are administered at relatively regular intervals, with no (therapeutically) significant interruptions. Naturally, minor interruptions may occur that do not affect the overall effectiveness of the present method, and indeed such aberrations are encompassed by the present disclosure. In a preferred example, and more arithmetically, the administration regimen is deemed to be continuous if the longest interval between two subsequent administrations is not more than 3.5 times as long as the average interval. Even more preferably said longest interval is not more than 2.5 times, most preferably not more than 1.5 times as long as the average interval.

In one example, the optional progestogenic component is administered via a non-oral route, for example using an Intra Uterine Device (IUD). In one example said IUD delivers the progestogenic component levonorgestrel. In one such example, the IUD is the Mirena^{®} IUD or the Levosert^{®} IUD.

A skilled person therefore appreciates that the estetrol component may be administered in conjunction with any other cosmetic or pharmaceutically active agents for alleviating or preventing hair loss known in the art.

In one example, the present therapeutic or cosmetic therapy employs oral, sublingual, buccal, or sublabial administration of the estetrol component. These latter three modes of administration offer the advantages that the estetrol component does not have to pass through the digestive system and avoids first-pass liver exposure. Furthermore, these modes of administration provide a rapid onset of action.

The term "sublingual" as used herein refers to the pharmacological route of administration by which the estetrol component diffuses into the blood through tissues under the tongue.

The term "buccal" as used herein refers to the pharmacological route of administration by which the estetrol component diffuses into the blood through tissues of the buccal vestibule, the area inside the mouth between the lining of cheek (the buccal mucosa) and the teeth / gums.

The term "sublabial" as used herein refers to the pharmacological route of administration by which the estetrol component is placed between the lip and the gingiva.

In the present therapeutic or cosmetic methods, the estetrol and progestogenic components may be administered in separate dosage units. However, it is also possible and indeed very convenient to combine these two components into a single dosage unit.

In the therapeutic or cosmetic methods according to the present disclosure the combination of the progestogenic and estetrol component is suitably administered continuously during a period of at least 10 days.

The disclosure may suitably be reduced to practice in the form of a variety of administration methods that are known to the person skilled in the art. Amongst these methods are the methods making use of monophasic preparations, which contain dosage units with a constant amount of the estetrol component and of the optional progestogenic component.

In the example of the disclosure where sequential administration of the progestogenic component is chosen, it is also possible and convenient to combine the components into a single dosage unit for the days when the two components are administered.

In another example of the disclosure, the therapy is administered to a postmenopausal subject.

In certain examples, the composition described herein, which is a composition intended for systemic administration, is used in conjunction with a cosmetic composition intended for local (i.e. cutaneous) administration which does not comprise an estrogen, preferably wherein said cosmetic composition does not comprise a hormone.

### Compositions

The estetrol component of the present disclosure encompasses substances selected from the group consisting of estetrol, esters of estetrol wherein the hydrogen atom of at least one of the hydroxyl groups has been substituted by an acyl radical of a hydrocarbon carboxylic, sulfonic acid or sulfamic acid of 1-25 carbon atoms; and combinations thereof. More preferably, the estetrol component is estetrol (including estetrol hydrates). Most preferably, the estetrol component contained in the dosage unit is estetrol monohydrate.

The estetrol component of the disclosure is used at a daily dose equivalent to from about 15 mg to about 25 mg of estetrol monohydrate. In other words, when the estetrol component is not estetrol monohydrate itself, the daily dose of the estetrol component is adjusted to yield a therapeutic effect equivalent to that of a daily dose of about 10 mg to about 25 mg of estetrol monohydrate, such as for example from about 14 mg to 16 mg estetrol, preferably estetrol monohydrate, or from about 19 mg to 21 mg estetrol, preferably estetrol monohydrate.

In a particularly preferred example of the disclosure the pharmaceutical composition according to disclosure is designed for daily administration, i.e. it represents a daily dosage unit.

In the case of oral administration, the oral dosage unit according to the disclosure is preferably a solid or semi-solid dosage unit (interchangeably indicated by the term "dosage form") such as tablets, capsules, cachets, pellets, pills, powders and granules. The term "solid or semi-solid dosage unit" also encompasses capsules that contain a liquid, e.g. an oil, in which the present estetrol component and/or the optional progestogenic component is dissolved or dispersed. Tablets and equivalent solid and semi-solid dosage units can suitably contain materials such as binders (e.g. hydroxypropylmethyl cellulose, polyvinyl pyrrolidone, other cellulosic materials and starch), diluents (e.g. lactose and other sugars, starch, dicalcium phosphate and cellulosic materials), disintegrating agents (e.g. starch polymers and cellulosic materials) and lubricating agents (e.g., stearates and talc). These tablets and equivalent solid dosage units may be prepared by wet granulation, e.g. using an aqueous solution or an organic solution, as well as by direct compression.

In the case of sublingual, buccal or sublabial administration, the pharmaceutical composition according to the disclosure is preferably an orodispersible dosage unit.

The term "orodispersible dosage unit" as used herein refers to a dosage unit that is designed to rapidly disintegrate in the oral cavity when it comes into contact with saliva and to disperse the estetrol component into the saliva so it may be absorbed through the mucosal lining of the oral cavity.

When the dosage unit is an orodispersible dosage unit, the rate of release of the estetrol component from the dosage unit can suitably be determined using the disintegration test according to Ph. Eur. 2.9.1 ("Disintegration of tablets and capsules") and USP <701> ("Disintegration"), for example using water as the disintegration medium. An orodispersible solid dosage unit of the present disclosure, when subjected to the aforementioned disintegration test, typically disintegrates within less than 5 minutes, more preferably within less than 2 minutes, still more preferably within less than 1,5 minutes, still more preferably within less than 1 minute, still more preferably within less than 45 seconds, and most preferably within less than 30 seconds.

In one example, the dosage unit comprises an estetrol component but no progestogenic component.

In one example, the disclosure provides an oral combined daily dosage unit comprising an estetrol component and a progestogenic component.

Preferably the progestogenic component is selected from the group consisting of progesterone, drospirenone, dydrogesterone, precursors of these progestogens and mixtures thereof.

In one example, the disclosure provides a combination composition comprising an estetrol component together with progesterone.

In one example, the disclosure provides a combination composition comprising an estetrol component together with drospirenone.

In one example, the disclosure provides a combination composition comprising an estetrol component together with dydrogesterone.

The estetrol and the progestogenic component can be present in separate oral dosage units or can be combined in the same oral dosage unit.

When the progestogenic component of the disclosure is drospirenone, it is preferably used at a daily dose of from 0.25 mg to 10 mg, even more preferably of from about 0.25 mg to about 4 mg, such as from about 1 mg to 4 mg, of from about 1 mg to 3 mg, or of from about 2.5 mg to 3.5 mg per day, preferably about 3 mg.

When the progestogenic component of the disclosure is dydrogesterone, it is preferably used at a daily dose of about 5 mg to about 10 mg, more preferably at a daily dose of about 5 mg.

When the progestogenic component of the disclosure is progesterone, it is preferably used at a daily dose of from 50 mg to 200 mg. In one example, progesterone is used at a daily dose of 50 mg to 100 mg when it is used continuously. In another example, progesterone is used at a daily dose of 100 mg to 200 mg when it is used sequentially, for example when it is administered during about 14 days every month.

When a different progestogenic component is used, the daily dose is adjusted such as to give the same pharmacological effect as a dose of 50 mg to 200 mg of progesterone.

In a preferred example of the disclosure, the composition combines the estetrol component and the optional progestogenic component into a single dosage unit, preferably a daily dosage unit. In a more preferred example of the disclosure, said combined daily dosage unit is an oral combined daily dosage unit.

In one example, the disclosure provides an oral combined daily dosage unit comprising an estetrol component and progesterone.

In one example, the disclosure provides an oral combined daily dosage unit comprising an estetrol component and drospirenone.

In one example, the disclosure provides an oral combined daily dosage unit comprising an estetrol component and dydrogesterone.

In a preferred example of the disclosure, an oral combined daily dosage unit combining estetrol at a daily dose of about 20 mg with progesterone at a daily dose of about 100 mg is provided.

In a preferred example, an oral combined daily dosage unit combining a daily dose from 14 mg to 16 mg estetrol or from 19 mg to 21 mg estetrol, preferably estetrol monohydrate, and of about 2.5 to 3.5 mg drospirenone is provided.

In another example of the disclosure, the estetrol component is administered to a patient who still has a uterus in conjunction with a Selective Estrogen Receptor Modulator (SERM), in particular in conjunction with bazedoxifene. Preferably bazedoxifene is administered at a daily dose of about 10 mg to 50 mg. More preferably, bazedoxifene is administered at a daily dose of about 20 mg.

In one example, the disclosure provides a therapy excluding progestogenic components.

The meaning of the term "modulating the average level" or "modulating the level" or "reducing the level" or "increasing the level" when used herein needs to be seen as encompassing respectively modulating, reducing or increasing the protein and/or mRNA expression level of the substance referred to as compared to a basal average level in a subject having a certain condition. The protein level in a subject can e.g. be analysed using standard techniques such as ELISA on a sample of the subject. The mRNA expression level in a subject can e.g. be analysed using standard techniques such as quantitative RT-PCR (Q-PCR) on a sample of the subject. In another example, the modulation involves increasing the protein or expression level

In certain preferred examples, the estetrol component is comprised in a solid dosage unit, preferably wherein each solid dosage comprises about 10 mg to 25 mg, such as about 14 mg to 21 mg or about 14 mg to 16 mg or about 19 mg to 21 mg or about 15 mg or about 20 mg of the estetrol component. In more preferred examples, the estetrol component is comprised in a tablet, caplet, or capsule, most preferably a tablet. In further examples, the solid dosage unit is formulated to correspond to a daily solid dosage unit. In yet further examples, the solid dosage unit is an oral dosage unit (i.e. a dosage unit which is intended to be swallowed by the subject). Hence, in certain examples the medical use or method of treatment comprises the daily ingestion of a solid dosage unit comprising an effective amount of estetrol. In certain examples, the effective amount of estetrol component is comprised in a single dosage unit which further comprises at least one pharmaceutically active agent which can be a progestin as defined herein elsewhere or can be an additional cosmetic or (pharmaceutically) active ingredient for preventing or treating hair loss.

In alternative examples, the solid dosage unit is suited for sublingual, buccal, and/or sublabial administration. In such examples, the solid dosage unit is able to rapidly release the estetrol component when contacted with an aqueous solvent such as saliva. Hence, in these examples the solid dosage unit is an orodispersible dosage unit which releases at least about 50%, preferably at least about 60%, more preferably at least about 70%, yet more preferably at least about 80%, most preferably more than about 80% of the estetrol component within about 5 minutes, preferably within about 3 minutes, more preferably within about 2.5 minutes, more preferably within about 90 seconds, most preferably within about 90 seconds. The term "orodispersible dosage unit" as used herein refers to a dosage unit that is designed to rapidly disintegrate in the oral cavity when it comes into contact with saliva and to disperse the estetrol component into the saliva so it may be absorbed through the mucosal lining of the oral cavity. A skilled person is aware of methods to determine the release rate of an estetrol component from a dosage unit. Non-limiting standardized tests generally accepted in the field include the disintegration test according to Ph. Eur. 2.9.1 ("Disintegration of tablets and capsules") and USP <701> ("Disintegration"), for example using water as the disintegration medium.

In certain examples, the estetrol component is comprised in an immediate release dosage unit or composition. In alternative examples, the estetrol component is comprised in a delayed release, sustained release, or controlled release dosage unit or composition. The terms "immediate release", "delayed release", and "sustained-release" or "controlled release" are clear to a person skilled in the art and are indicative for the release profile of a pharmaceutical composition. In immediate release the pharmaceutical composition is about immediately released from a dosage unit to a body of a subject or patient. In delayed release dosage units, the pharmaceutical composition is delivered in the body with a delay after administration. In sustained release or controlled release dosage units, the dosage unit is designed to release a pharmaceutical composition at a predetermined rate in order to maintain a constant drug concentration for a specific period of time. The release profile of a dosage unit can be assessed as described in the major pharmacopeias. For example, immediate release is defined by the European Medicines Agency as dissolution of at least 75% of the active substance within 45 minutes (European Pharmacopeia (Ph. Eur.) 9th edition). However, it is in addition trivial to a person skilled in the art that suitable tests and time windows may vary depending on therapeutic ranges, solubility and permeability factors of the drug substance.

In alternative examples, the modes of administration that may be considered in the context of the present disclosure are not particularly limited as long as they result in systemic bioavailability. These include but are not limited to: oral, rectal, bronchial, nasal, buccal, sublingual, vaginal, transdermal, subcutaneous, intrauterine or parenteral administration, or in a form suitable for administration by inhalation or insufflation, including powders and liquid aerosol administration. In preferred examples, the mode of administration is oral administration. It is understood that oral administration includes administration by oral ingestion, but additionally includes administration modes such as buccal and sublingual administration, optionally by means of an orodispersible dosage unit such as but not limited to an orodispersible tablet. In all cases, the goal is to achieve a similar effective dose of estetrol in blood plasma (e.g. similar AUC and/or Cmax values) as for using the oral dosage unit.

In certain examples, the estetrol component is formulated into a unit dosage unit, including but not limited to hard capsules, soft capsules, tablets, coated tablets such as lacquered tablets or sugar-coated tablets, granules, aqueous or oily solutions, syrups, emulsions, suspensions, inhalants or suppositories, which may be provided in any suitable packaging means known in the art, non-limiting examples being troches, sachets, pouches, bottles, films, sprays, microcapsules, implants, rods or blister packs. In examples wherein the effective amount of an estetrol component is administered by means of oral administration, the oral dosage unit according to the disclosure is preferably a solid or semi-solid dosage unit such as tablets, capsules, cachets, pellets, pills, powders and granules. A particularly preferred composition comprises estetrol monohydrate in the dosages disclosed herein, in combination with excipients suitable for a solid or semi-solid dosage unit.

The term "solid or semi-solid dosage unit" also encompasses capsules that contain a liquid, e.g. an oil, in which the present estetrol component and/or the optional progestogenic component is dissolved or dispersed. Tablets and equivalent solid and semi-solid dosage units can suitably contain materials such as binders (e.g. hydroxypropylmethyl cellulose, polyvinyl pyrrolidone (povidone, PVP), other cellulosic materials and starch), diluents (e.g. lactose (monohydrate) and other sugars, starch (e.g. Maize starch), dicalcium phosphate and cellulosic materials), disintegrating agents (e.g. starch polymers and cellulosic materials (e.g. sodium starch glycolate) and lubricating agents (e.g., (magnesium) stearates and talc). These tablets and equivalent solid dosage units may be prepared by any suitable means, which have been described in detail in the art (e.g. Kaur, Processing technologies for pharmaceutical tablets: A review, Int Res J Pharm, 2012). Non-limiting examples of processing the estetrol component when manufacturing the dosage unit include wet granulation, e.g. using an aqueous solution or an organic solution, direct compression, 3D printing, or by coating carrier particles with the estetrol component using an organic or inorganic solvent.

It is evident that a further aspect of the disclosure relates to pharmaceutical or cosmetic compositions comprising an effective amount of the estetrol component for use in any of the medical indications disclosed herein. The terms "formulation" or "composition" may be used interchangeably herein. In any of the examples concerning one or more of the pharmaceutical or cosmetic composition described herein, it is evident that said composition may comprise one or more pharmaceutically or cosmetically acceptable carriers (i.e. excipients). The term "pharmaceutically acceptable" or "cosmetically acceptable" as used herein is consistent with the art and means compatible with the other ingredients of a pharmaceutical or cosmetic composition and not deleterious to the recipient thereof. In a particularly preferred example of the disclosure the pharmaceutical composition according to disclosure is designed for daily administration, i.e. it represents a daily dosage unit. The excipients that may be used in the pharmaceutical composition is not particularly limited and may therefore be one or more excipients selected from the group consisting of: an active pharmaceutical ingredient excipients, binder excipients, carrier excipients, co-processed excipients, coating system excipients, controlled release excipients, diluent excipients, disintegrant excipients, dry powder inhalation excipients, effervescent system excipients, emulsifier excipients, lipid excipients, lubricant excipients, modified release excipients, penetration enhancer excipients, permeation enhancer excipients, pH modifier excipients, plasticiser excipients, preservative excipients, preservative excipients, solubilizer excipients, solvent excipients, sustained release excipients, sweetener excipients, taste making excipients, thickener excipients, viscosity modifier excipients, filler excipients, compaction excipients, dry granulation excipients, hot melt extrusion excipients, wet granulation excipients, rapid release agent excipients, increased bioavailability excipients, dispersion excipients, solubility enhancement excipients, stabiliser excipients, capsule filling excipients, or any combination hereof. A skilled person is aware that use of such media and agents for pharmaceutical active substances is common practice and incorporation of these excipients is hence well known in the art. It is evident that all of the used ingredients should be non-toxic in the concentration contained in the final pharmaceutical composition and should not negatively interfere with the activity of the estetrol component, said estetrol component preferably being present in the pharmaceutical composition as the predominant pharmaceutically active ingredient. In certain examples, more than one excipient which a skilled person would classify as belonging to the same group of excipients is added to the pharmaceutical composition. In further examples, more than one excipient wherein the different excipients belong to different groups is added to the pharmaceutical composition. In certain examples, the excipients may fulfil more than one function and/or be classified by a skilled person as belonging to different groups or classes of excipients.

The compositions envisaged by the present disclosure may comprise further skin active components capable of providing a skin care benefit. The skin care benefit may include but is not limited to benefits related to cosmetic appearance of the skin. The further skin active component may provide an immediate and short lived (i.e. acute) benefit, and/or a long term and long lasting (i.e. chronic) benefit.

The compositions envisaged by the present disclosure may comprise further hair loss preventing or hair care promoting active components capable of providing a hair care benefit known in the art. The hair care benefit may include but is not limited to benefits related to cosmetic appearance of the hair, as well as growth thereof. The further hair care active component may provide an immediate and short lived (i.e. acute) benefit, and/or a long term and long lasting (i.e. chronic) benefit.

While the disclosure has been described in conjunction with specific examples thereof, it is evident that many alternatives, modifications, and variations will be apparent to those skilled in the art in light of the foregoing description. Accordingly, it is intended to embrace all such alternatives, modifications, and variations.

The following specific experimental examples are provided in support of the disclosure.

### Examples

### Example 1: Tissue distribution of estetrol after oral administration in a rat model

Tissue distribution was determined in female rats (partially pigmented and non-pigmented) using [¹⁴C]-estetrol.

Following a single oral dose of [¹⁴C]-estetrol to female non-pigmented rats, concentrations of radioactivity were generally maximal at the first time point of 0.5 hours with the greatest concentration measured in the liver (12.5 µg equivalents/g). Radioactivity was eliminated rapidly such that by 24 hours after dosing, in addition to components of the GI tract and bladder, only the adrenal gland, kidney, liver, pancreas, salivary gland and thyroid gland were quantifiable. Following a single oral dose of [¹⁴C]-estetrol to female partially pigmented rats, concentrations of radioactivity were generally maximal at the first time point of 0.5 hours after oral dosing with the greatest concentration measured in the liver (13.7 µg equivalents/g). Radioactivity was again eliminated quickly, with the majority of tissues BLQ (<0.076 µg equivalents/g) by 24 hours after dose administration. By 7 days after dosing, all analysed tissues, with the exception of the liver (0.410 µg equivalents/g), were at or below the limit of quantification, including melanin-containing structures (uveal tract, pigmented skin).

### Protocol - tissue distribution study in non-pigmented rats

A group of seven female Sprague-Dawley rats each received a single oral dose of [¹⁴C]-estetrol. At each selected time (see below) one animal was killed (by overdose of CO2) followed by immediate snap freezing in hexane/solid CO2. The carcasses were retained for examination of the tissue distribution of radioactivity using the technique of quantitative whole body autoradiography (QWBA). Times for analysis were: 0.5, 1, 2, 5, 8, 24 and 48 hours.

Following completion of whole-body autoradiography sectioning, remaining carcasses were disposed of as waste.

Distribution of radioactivity in tissues was determined and quantified using the Fuji FLA-5100 fluorescent image analyser and associated Tina and SeeScan software. Tissue concentrations of radioactivity were determined using calibrated autoradiographic microscales produced by GE Healthcare. A standard curve was produced using the microscales from which tissue concentrations of radioactivity were determined (nCi/g). These values were then converted into µg equivalents/g using the specific activity of the dose formulation.

### Protocol - tissue distribution study in pigmented rats

A group of six female Lister Hooded rats each received a single oral dose of [¹⁴C]-estetrol. At each selected time (see below) one animal was killed (by overdose of CO2) followed by immediate snap freezing in hexane/solid CO2. The carcasses were retained for examination of the tissue distribution of radioactivity using the technique of quantitative whole body autoradiography (QWBA).

Times for analysis were: (0.5, 8, and 24 hours, 7, 14 and 35 days).

Frozen carcasses were subjected to whole-body autoradiography.

### Results in non-pigmented rats (focused on the skin)

Where concentrations are reported, radioactivity was assumed to be associated with [¹⁴C]-estetrol or any metabolic products derived from [¹⁴C]-estetrol. The specific activities of the dosed test compounds were used for the calculation of concentrations in all cases. Recoveries of radioactivity are reported as percentage of administered radioactivity (% dose). Radioactivity concentrations are also reported as µg equivalents/g.

Concentrations of radioactivity in tissues (skin only here) and tissue:blood ratios obtained from female Sprague-Dawley rats following a single oral dose of [¹⁴C]-estetrol are presented in Tables 1 and 2, respectively.

**Table 1: Concentration of radioactivity (expressed as microgram equivalents/gram) in tissues following a single oral administration of [¹⁴C]-estetrol to female non-pigmented rats at a nominal dose level of 15 mg/kg.**

| Tissue | Rat 07F 0.5 hours | Rat 08F 1 hour | Rat 09F 2 hours | Rat 10F 5 hours | Rat 11F 8 hours | Rat 12F 24 hours | Rat 13F 48 hours |
|---|---|---|---|---|---|---|---|
| Skin: Non-pigmented | 2.59 | 0.771 | 0.465 | 0.189 | 0.361 | BLQ | BLQ |

**Table 2: Tissue:Blood ratios determined following a single oral administration of [¹⁴C]-estetrol to female non-pigmented rats at a nominal dose level of 15 mg/kg.**

| Tissue | Rat 07F 0.5 hours | Rat 08F 1 hour | Rat 09F 2 hours | Rat 10F 5 hours | Rat 11F 8 hours | Rat 12F 24 hours | Rat 13F 48 hours |
|---|---|---|---|---|---|---|---|
| Skin: Non-pigmented | 1.05 | 0.99 | 1.72 | 1.66 | 1.77 | NC | NC |

### Results in pigmented rats (focused on the skin)

Concentrations of radioactivity in tissues and tissue: blood ratios obtained from female Lister hooded rats following a single oral dose of [¹⁴C]-Estetrol are presented in Tables 3 and 4, respectively.

**Table 3: Concentration of radioactivity (expressed as microgram equivalents/gram) in tissues following a single oral administration of [¹⁴C]-estetrol to female partially pigmented rats at a nominal dose level of 15 mg/kg.**

| Tissue | Rat 14F 0.5 hours | Rat 15F 8 hours | Rat 16F 24 hours | Rat 17F 7 days | Rat 18F 14 days | Rat 19F 35 days |
|---|---|---|---|---|---|---|
| Skin: Non-pigmented | 1.89 | 0.090 | BLQ | BLQ | BLQ | BLQ |
| Skin: Pigmented | 2.85 | 0.161 | 0.113 | BLQ | BLQ | BLQ |

**Table 4: Tissue:Blood ratios determined following a single oral administration of [¹⁴C]-estetrol to female partially pigmented rats at a nominal dose level of 15 mg/kg.**

| Tissue | Rat 14F 0.5 hours | Rat 15F 8 hours | Rat 16F 24 hours | Rat 17F 7 days | Rat 18F 14 days | Rat 19F 35 days |
|---|---|---|---|---|---|---|
| Skin: Non-pigmented | 0.71 | 0.56 | NC | NC | NC | NC |
| Skin: Pigmented | **1.07** | 1.01 | NC | NC | NC | NC |

Total amounts of radioactivity in selected tissues/organs, expressed as % dose/tissue, tissue % body weights taken from Caster [2], are presented in Table 5.

**Table 5: Total amounts of radioactivity in select tissues following a single oral administration of [¹⁴C]-estetrol to female partially pigmented rats at a nominal dose level of 15 mg/kg.**

| Results expressed as % dose administered/tissue | | | | | | |
|---|---|---|---|---|---|---|
| Tissue | Rat 14F 0.5 hours | Rat 15F 8 hours | Rat 16F 24 hours | Rat 17F 7 days | Rat 18F 14 days | Rat 19F 35 days |
| Skin (non-pigmented) | 1.136 | 0.059 | <0.041 | <0.043 | <0.038 | <0.041 |
| Skin (pigmented) | 1.714 | 0.099 | 0.061 | <0.043 | <0.038 | <0.041 |

To conclude: At the first sampling time (0.5 hours), radioactivity, based on total dose/tissue, was greater than 1% of the administered dose in pigmented and non-pigmented skin (1.7% and 1.1 % respectively). As is clear from the results, significant E4 concentrations are achieved in the skin after oral administration, indicating that the estetrol was adequately reaching the skin to be therapeutically effective.

### Example 2: Effects of E4 in vitro on keratinocytes and fibroblasts as the two main cell types in the skin

The aim of the study is to evaluate the effects of estetrol on migration and proliferation capacities of human epidermal keratinocytes and of human dermal fibroblasts in 2D cultures and to directly compare the effects of this estrogen to estradiol as another reference estrogen (E2).

The study consists in an in vitro assay aiming to evaluate the properties of E4 and E2 at 4 concentrations each on keratinocytes and fibroblasts. In this assay, the measurement of the colonization of a standard acellular surface generated from a confluent monolayer of cells using a dedicated culture device (IBIDI chamber) is performed. The assay is performed in absence and in presence of a proliferation inhibitor to focus on cell proliferation and migration or on migration only, respectively.

### Methodology

### Cellular model - Normal Human Epidermal Keratinocytes (NHEKs)

The study was performed using cultures of Normal Human foreskin-derived Epidermal Keratinocytes (NHEKs, Lonza). The cells were grown in serum free Epilife medium (ThermoFisher Scientific) supplemented with Human Keratinocyte Growth Supplement (HKGS, ThermoFisher Scientific) and antibiotics. The cells were maintained in a humidified incubator at 37°C with a 5% CO2 atmosphere.

### Cellular model - Normal Human Dermal Fibroblasts (NHDFs)

Normal Human forskin-derived Dermal Fibroblats (NHDFs, ATCC) cells were grown in phenol-free Dulbecco's Modified Eagle Medium (DMEM, ThermoFisher Scientific) supplemented with 10% of Foetal Bovine Serum (FBS, ThermoFisher Scientific), L-glutamine and antibiotics. The cells were maintained in a humidified incubator at 37°C with a 5% CO2 atmosphere.

### Assay and analysis

Cells were plated in 2-well silicone inserts with a defined cell free gap (IBIDI chambers; IBIDI GmbH, 80209) that was previously inserted on 12-well culture plates. This culture device allows to create a standardized 500 µm ± 100 µm cell free gap for monitoring cell colonization. 24h after seeding, when the culture reached high confluency, cells in the insert were treated, or not, with mitomycin C (MMC) at 10 µg/ml during 2h. After these 2 hours, the insert was removed, and cells were treated with E2 or E4 in presence or not of MMC for additional 2h. Then, if appropriate, the culture medium was removed and cells were treated with E2 or E4 for 22h, in absence of MMC.

For the assay, keratinocytes were plated in Epilife medium without HKGS and all the treatments were performed in this medium. Fibroblasts were plated in DMEM medium containing only 2,5 % of FBS and the treatments were performed in absence of FBS.

E2 and E4 were applied at 4 concentrations each (10⁻⁷M, 10⁻⁸M, 10⁻⁹M, 10⁻¹⁰M). In parallel, cells were treated with a positive control (HKGS 1% for human keratinocytes and PDGF-BB at 10 ng/ml for human fibroblasts).

To monitor cell colonization and to evaluate the gap closure rate, pictures of the gap were taken after different times: at time 0h, corresponding to the beginning of the treatment with E2 or E4 and to the removal of the culture insert, at time 6h and at time 24h (corresponding to the end of the treatments with E2 or E4). Pictures were taken with a microscope Zeiss (Axiover 25 - 10x objective) and with a camera Invenio2Elll (DeltaPix - Lux-Optic). The colonized areas were quantified from the pictures by the use of the WimAsis platform - WimScratch software. For each insert, 3 pictures were taken at each time. As each condition was performed in triplicates, it means a total of 9 pictures per condition and per time point of acquisition

### Results

### Normal Human Epidermal Keratinocytes (NHEK)

The treatment with HKGS used as positive reference allowed to significantly increase the colonized area, and this in presence and in absence of MMC, validating the test.

In presence of the proliferation inhibitor (MMC), E4 at 10⁻⁷M and 10⁻¹⁰M significantly increased the colonization of the acellular area in comparison to ethanol control after 24h of treatment, indicating a stimulation of cell migration (Figure 1). In absence of MMC at 6H, E4 at 10⁻⁷M and 10⁻¹⁰M significantly increased the colonization of the acellular area in comparison to the ethanol control (Figure 2).

In the presence or absence of the proliferation inhibitor (MMC), E2 was not able to significantly increase the colonization of the cell-free area in comparison to the ethanol control (Figure 3 and 4). At concentrations between 10⁻⁷M and 10⁻⁹ M, it seems even to have an inhibitor effect on the gap closure, after 24h of treatment. This inhibition is significant only for the concentration of 10⁻⁸M.

### Normal Human Dermal Fibroblasts (NHDFs)

The treatment with PDGF-BB at 10 ng/ml used as positive reference significantly increases the colonized area, both in presence and absence of MMC, validating the assay.

In absence of proliferation inhibitor (MMC), E4 was not able to significantly increase the colonization of the cell-free area (Figure 6). But, in presence of MMC, at 24H, E4 at 10⁻⁷M and 10⁻⁸M allowed to significantly increase the colonization of the acellular area in comparison to ethanol control (Figure 5).

In presence of the proliferation inhibitor (MMC), E2 was not able to significantly increase the colonization of the cell-free gap (Figure 7). But, in absence of MMC at 24H, E2 at 10⁻⁷M, 10⁻⁸M and 10⁻¹⁰M increased the colonization of the acellular area in comparison to the ethanol control (Figure 8).

### Conclusion

In this study, we noticed that E4 at 10⁻⁷M and 10⁻¹⁰M significantly increases the surface colonized over time by keratinocytes in culture. The effects were significant after 6h and 24h of treatment respectively in absence and presence of MMC. E4 might thus have a stimulatory effect on cell proliferation as observed after 6h of treatment but might induce as well keratinocyte migration after a certain delay, given the difference of colonized surface observed at 24h in presence of the proliferation inhibitor. In presence of E2, no significant effect on keratinocyte proliferation or migration could be observed.

E4 and E2 demonstrated significant activity on fibroblast cultures after 24h of treatment, E4 rather acting on cell migration (at 10⁻⁷M and 10⁻⁸M) while E2 seemed to have a specific effect on fibroblasts proliferation (at 10⁻⁷M, 10⁻⁸M and 10⁻¹⁰M). The results are summarized in Table 6 below:

**Table 6: Activity of estetrol (E4) or estradiol (E2) on keratinocytes (NHEK) and fibroblasts (NHDF).**

| | | NHEK | | NHDF | |
|---|---|---|---|---|---|
| | | Proliferation & Migration | Migration (+MMC) | Proliferation & Migration | Migration (+MMC) |
| Estetrol (E4) | 10⁻⁷ M | ↗**6H** | **↗ 24H** | - | ↗ **24H** |
| | 10⁻⁸ M | - | **-** | - | **↗↗ 24H** |
| | 10⁻⁹ M | - | **-** | - | **-** |
| | 10⁻¹⁰ M | ↗ **6H** | **↗ 24H** | - | - |
| Beta-estradiol (E2) | 10⁻⁷ M | - | **-** | ↗ **24H** | - |
| | 10⁻⁸ M | - | **-** | ↗ **24H** | - |
| | 10⁻⁹ M | | | **-** | - |
| | 10⁻¹⁰ M | - | - | **↗ 24H** | - |

### Example 3: Effects of estetrol (E4) on keratinocytes, fibroblasts and immune cells in a wound healing in vitro model

The acute wound response involves a range of local activated and peripherally recruited cell types. This pilot study investigated the effects of estetrol (E4) on multiple wound-relevant cell types (keratinocytes, fibroblasts and immune cells) with a direct comparison with the effects of estradiol (E2).

### Keratinocytes and fibroblasts experiments

### Protocol

Human skin was transferred to the laboratory in holding media (DMEM containing 4% penicillin-streptomycin and Amphotericin B solution) within 30 minutes of collection. Once defatted, the skin was rinsed in HBSS with 4% PSA followed by DPBS without antibiotics, then cut into thin strips and floated on 0.2% Dispase II in DPBS overnight at 4°C, followed by manual separation of the epidermis from the dermis. Primary human epidermal keratinocytes and primary human dermal fibroblasts were then isolated.

Confluent wells of both primary keratinocytes and fibroblasts were subjected to a highly validated scratch wounding procedure (using a 1 mL sterile filter tip). Post-scratching, cells were rinsed with DPBS and cell-specific media containing treatments added to each well. Cells were imaged on a ptychographic live cell imaging platform (Livecyte 2TM, Phasefocus) for 24-48 hours. Automated tracking of individual unlabeled cells will be combined with 3D cell measurements to quantify a range of wound-relevant cell parameters including scratch closure rate.

Dose range finding experiments were conducted with E4 (10⁻¹⁰M to 10⁻⁶M) and E2 with and without Mitomycin C (MMC, to inhibit proliferation) using vehicle as negative controls.

### Fibroblasts - results

Using the widely validated in vitro fibroblast monolayer scratch assay, we demonstrated that E4 promotes in vitro wound closure to a similar extent as E2, across both mouse and human fibroblasts (Figure 9A and B respectively). The assay was performed in the phenol red-free cell culture media containing charcoal stripped fetal bovine serum, using fibroblasts isolated from female C57BI/6 mice and from surplus abdominal skin donated by three female donors aged 20, 49 and 57.

### Keratinocytes - results

The data show that the impact of estrogens (E4 and E2) on keratinocyte migration was dependent on the % of HKGS (Human Keratinocyte Growth Supplement) used in the culture media. However, in culture condition with 15% HKGS, the impact of E4 (10⁻¹⁰M and 10⁻⁷M) on keratinocytes seems higher than the impact of E2 (Figure 10).

### Immune cells experiment

Immune cell polarization was evaluated in vitro using markers for M1/TH-1 (NOS2) and M2/TH-2 (ARG1) phenotype with human monocytes and mouse monocytes (BMDM bone marrow derived monocytes).

### Protocol

Human blood was collected under national ethical approval with full informed written patient consent. Freshly isolated human blood was collected in EDTA vacutainer tubes, diluted 1:1 in PBS and gently layered onto histopaque (Merck). Tubes were immediately centrifuged at 100 x g for 30 minutes and the whitish buffy layer formed at the histopaque interface aspirated and washed (centrifuged 100 x g, 10 minutes) twice with sterile PBS. Untouched classical (CD14++, CD16- ) monocytes were isolated from the washed buffy layer cells using the Miltenyi Classical Monocyte Isolation Kit, LS columns and a MidiMACs separator (Miltenyi). Isolated cells were seeded into tissue culture plates and stimulated to differentiate using PMA.

An experiment was conducted to evaluate the anti-inflammatory effects of E4 or vehicle (negative control). qPCR methodology was used to assess the effects of E2 and E4 on immune cell polarization using well validated marker of pro-inflammatory (NOS2 for MI/TH-1 phenotype) and anti-inflammatory (ARG1 for ME/TH-2 phenotype) activity.

### Results

E4 showed anti-inflammatory effects, promoting mouse BMDM (bone marrow derived monocytes) or human monocytes switch from subtype M1 (pro-inflammatory) to subtype M2 (healing). The results are depicted in Figure 11 A and B respectively.

### Example 4: Effects of E4 on keratinocytes and fibroblasts gene expression in a wound healing in vitro model

The healing properties of E4 were investigated at the transcriptional level by studying gene expression changes by RT-qPCR. 84 genes qPCR arrays focusing on the wound healing process were used. These arrays contain genes important for the three phases of wound healing process, including extra-cellular matrix (ECM) remodeling factors, inflammatory cytokines and chemokines as well as growth factors and major signaling molecules. The changes in gene expression were interpreted in a dermo cosmetic context in order to highlight the potential benefits of the E4 towards wound healing.

### Cell treatment

After 24h seeded on 25 cm² culture flasks (T25), cells were treated with E4 during 15h. The culture conditions were the same as the one used for the scratch test (see Example 2) and cells were treated with E4 in absence of FBS/HKGS. The cell density used for gene expression assay was lower than the ones used for the scratch tests: 15 800 cells/cm² for gene expression assay vs 113 636 cell/cm² for keratinocytes and 100 000 cells/cm² for fibroblasts in culture inserts for wound closure assay. Indeed, gene expression analysis are usually performed on under-confluent cells (during logarithmic growth phase).

### Total RNA extraction

At the end of the 15h treatment with E4 10⁻⁷M or ethanol 0.1% used as control, total RNAs were extracted using the Qiagen RNeasy kit (Qiagen). Cells were rinsed with cold PBS and lysed in the ad hoc buffer provided in the kit. Extraction was performed according to the supplier's recommendations. The collected RNAs were stored at -80°C.

### RNA integrity analysis

The RNA concentration was determined by spectrophotometric measurement (QlAxpert, Qiagen) and the RNA quality was analyzed by capillarity electrophoresis (Agilent Bioanalyzer 2100 - Agilent RNA 6000 Nano Kit).

### cDNA synthesis

Reverse transcription was performed with kit "RT2 First Strand Kit" (Qiagen) from total RNA according to the manufacturer's instructions.

### Quantitative PCR using RT2 Profiler PCR arrays

The qPCR method was used to quantify the expression changes of specific markers in the cDNA populations. The arrays "RT2 Profiler PCR Array human wound healing" containing specific primers for 84 target genes.

The amplification of target genes was performed following the supplier's recommendations thanks to a "HotStart Taq DNA Polymerase" and to the use of SYBR Green as detection method. The SYBR Green is a marker emitting fluorescence once inserted between the paired bases of nucleic acids. The fluorescence intensity is thus directly proportional to the quantity of produced amplicons.

The qPCRs were run using the Quantstudio7 Real-Time PCR System (Applied Biosystems) and its software (QuantStudio real time PCR Software v1.3. software, Applied Biosystems).

The data obtained for E4 treated conditions were compared to the ethanol control in both cell types. The threshold cycles (Ct) values were normalized to the mean of Ct of several housekeeping genes present on the array (indicated in the table below). The maximum Ct cut-off value was fixed at 36 cycles meaning that Ct values above 36 were not considered in the analysis.

### Results

Treatment of human keratinocytes (Table 7) or fibroblasts (Table 8) with 10⁻⁷M E4 for 15 hours induced certain interesting pathways involved in the different phases of the wound healing process.

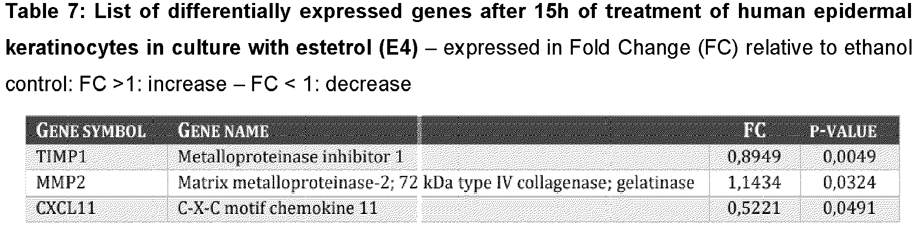

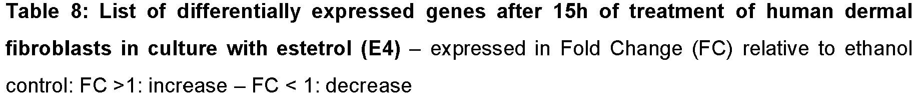

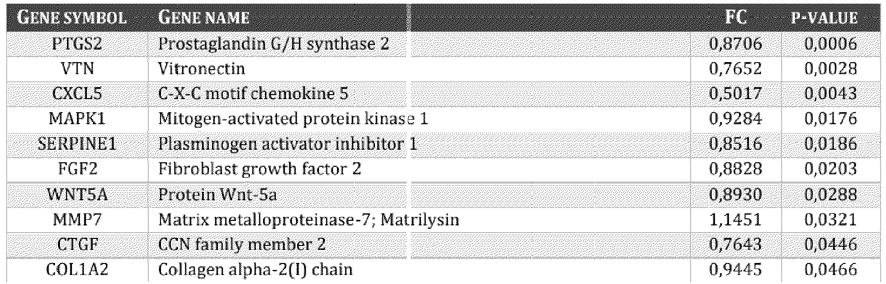

Although few in number and low in amplitude, these variations in gene expression (compared to ethanol control) suggest that E4 could promote the switch between the inflammatory phase and the proliferative/remodeling phases through down-regulation of pro-inflammatory factors and stimulation of the coupled MMP/TIMP system.

Treatment of human fibroblasts or keratinocytes with 10⁻⁷M E4 for 15 hours significantly decreased expression of the chemokines, CXCL5 and CXCL11, cyclo-oxygenase 2 (PTGS2) and fibroblast growth factor 2 (FGF2), reinforcing a switch to proliferative phase.

### Example 5: Evidence of the antioxidant property of E4

The objective of the study was to investigate the generation of reactive oxygen species (ROS) induced by the test item, estetrol (E4), in a mouse lymphoma cell line L5178Y TK^{+/-}.

### Protocol

L5178Y TK^{+/-} cells in logarithmic phase of growth were plated in black 96 well cell culture plates at 1 x 10⁴ cells in 90 µL of cell culture media. Controls and test item in triplicate were applied to the cells as a 10x concentrate in 10 µL of a 10% ethanol solution in PBS (final concentration of ethanol on plate was 1%). Cells were incubated for 3 hours at 37°C, 5% CO2, and the generation of ROS was evaluated by fluorescence with a ROS reagent: the ROS reagent was freshly prepared by dissolving one ampoule of reagent (50 µg) in 70 µL DMSO and further diluting this with 2.1 mL of pre-warmed PBS. 10 µL of ROS reagent was added to each well and the plate were shaken for 30 seconds in the dark. The plates were incubated approximately at 37°C, 5% CO2 for 1 h 30 min and fluorescence was read on a plate scanner with excitation at 492 nm and emission at 520 nm.

Three experiments were performed with the negative control lactate (known not to induce ROS) and the positive control hydrogen peroxide (known to increase ROS in cells) at concentrations up to 100 µM and with the test item at concentrations of 1, 10, 50, 100, 1000 and 3000 µM. The results for ROS induction for the negative control, positive control and test item-treated cells were compared to those obtained with the untreated vehicle control.

### Results

The negative control lactate did not induce ROS in any experiment at concentrations up to 100 µM when compared to the vehicle control. The positive control hydrogen peroxide showed a tendency to increase the levels of ROS after 3 h exposure compared to the vehicle control.

E4 up to 3000 µM caused a dose-dependent decrease in the levels of ROS detected in the L5178Y TK^{+/-} cells, whilst lactate up to 100 µM had no effect on ROS induction after 3 h exposure.

### Conclusion

E4 caused a dose-dependent decrease in ROS in all three experiments with the highest effect at the highest concentration tested, 3000 µM, suggesting E4 acted as an antioxidant in this study.

### Example 6: Hair follicle organ culture (ex vivo model)

12-44 full-length frontotemporal hair follicles (HF) were obtained from four human female donors >50 years old. Donors were caucasian female subjects of 59, 62, 59 and 67 years old, respectively (Donor 1: 12 temporal anagen HFs; Donor 2: 29; Donor 3: 44; Donor 4: 32 ). After microdissection from skin as described (Edelkamp et al., Methods Mol Biol, 2020, 2154:105-119; and Langan et al., Exp Dermatol, 2015, Dec;24(12):903-1), HFs were placed in adequate culture medium and allowed a 24h rest period to allow them to recover from the trauma of microdissection. Test substance (either vehicle (0.3% EtOH) or E4 at concentrations of 30µM, 3µM or 300 nM) was added to the medium at the end of the 24h. Total culturing time was 5-7 days depending on the studied parameters and the experimental question. The experimental design for the hair follicle organ culture experiment is depicted in Figure 12. Longer term culture experiments may be used to analyze the capacity to delay the onset of catagen.

This model allows to assess if E4 treatment is able to prolong anagen duration and/or to delay catagen initiation. Clinically, hair loss is associated with a reduction of HFs in anagen or with a shortening of anagen duration. Promoting anagen might thus limit hair loss.

The following parameters were investigated:

### • Hair shaft elongation ex vivo (digital brightfield microscope)

This parameter reflects HF growth rate thereby correlating with HF elongation or hair shaft production.

To determine the HF length, each HF was measured from the end of the bulb connective tissue sheath to the end of the distal outer root sheath (ORS), using a digital light microscope at 50 x magnification (VHX 900 Keyence Corporation, Osaka, Japan) and the affiliated software (Edelkamp et al., Methods Mol Biol, 2020, 2154:105-119; and Langan et al., Exp Dermatol, 2015, Dec;24(12):903-1). Measurements were obtained at baseline, after 24h rest period and subsequently every 24-48h during the entire duration of the experiment. Elongation is expressed as millimeters of growth and/or as percentage elongation from the baseline measurement.

### • Hair matrix keratinocytes proliferation and apoptosis

Germinative hair matrix keratinocytes proliferate mostly during anagen. Their proliferation is dramatically reduced during the anagen/catagen transition, and absent in late catagen (Langan et al., Exp Dermatol, 2015). Apoptosis of hair matrix keratinocytes is used as an additional indicator of anagen/catagen transition.

Proliferation was assessed by quantification of the percentage of hair matrix keratinocytes in a clearly specified area (germinative hair matrix keratinocytes, below the Auber's line) that were positive for the proliferation marker Ki67 (or another proliferation marker such as BrdU or EdU), whereas apoptosis was assessed using TUNEL (TdT-mediated dUTP -biotin nick-end labelling) immunofluorescence (or another apoptosis marker such as activated caspase-3) in the same keratinocyte population (below the Auber's line) and in the precortical hair matrix keratincoytes (above the Auber's line) (Langan et al., Exp Dermatol 2015) (Figure 13).

### • Macroscopic and microscopic hair cycle staging and hair cycle score

Hair cycle staging and calculation of hair cycle score is indicative of hair cycle progression and anagen-catagen transition (Langan et al., Exp Dermatol, 2015). Hair cycle staging and hair cycle score was performed at the end of the culture. The hair cycle stage of each HF was determined according to several established macroscopic and microscopic parameters (Langan et al Exp Dermatol, 2015). For macroscopic hair cycle stage, bright field images of the living HFs were taken with a digital microscope (Keyence VHX 900). Microscopic hair cycle staging was determined using Ki 67 /TUNEL immunohistology and Masson Fontana histochemistry. Hair cycle staging was analyzed by calculating the percentage of HFs in each hair cycle stage. Hair cycle score was determined by giving an arbitrary score to each hair cycle stage as following: anagen: 100, early catagen: 200, mid catagen: 300, late catagen: 400, dystrophic 500, with a higher score being indicative of a higher number of HFs in catagen.

| **Hair cycle stage** | **Anagen** | **Early catagen** | **Mid catagen** | **Late catagen** | **Dystrophic (anagen/catagen)** |
|---|---|---|---|---|---|
| Hair bulb shape | Rounded | Bullet-like | Slightly triangulated, swelling | Thinned and elongated | Swollen bulb, separation of the epithelial from the mesenchymal tissue, spongiosis in the ORS |
| Hair matrix shape and thickness | Thick, closed hair matrix | Thinner, opening of hair matrix | Very thin, shortened | Very thin, slightly withdrawn | As anagen or catagen |
| Dermal papilla (DP) shape | Almond-shaped | More rounded | Densely packed, and rounded | Very densely packed and rounded | As anagen or catagen |
| Accumulation fibroblasts in the dermal papilla stalk | no | yes | yes | yes | As anagen or catagen |
| Pigmentation | Very pigmented, starting at the level of Auber's line and expand to half of hair matrix | Less pigmented, starting at the level of Auber's line or above dermal papilla | Less pigmented, starling above dermal papilla | Less pigmented, starling at the level of club hair | Appearance of melanin clumping |
| Germinative hair matrix proliferation | Prominent below the Auber's line | Decrease in the number of proliferating cells below the Auber's line | No proliferation below the Auber's line | No proliferation below the Auber's line | As anagen or catagen |
| Hair matrix apoptosis | No apoptosis in the hair matrix | Little apoptosis may be observed in | Apoptosis in the hair matrix and epithelial strand | Apoptosis in the epithelial strand | As anagen or catagen |
| | | the hair matrix | | | |

The anagen and early catagen phases, as well as a representative image of a dystrophic hair follicle are depicted in Figure 14.

### • Lactate dehydrogenase (LDH) release into the medium

LDH is released from the cytoplasm of damaged cells into the culture medium. Therefore, the cytotoxicity of agents can be estimated based on the activity of LDH present in the culture medium using a colorimetric assay (Cytotoxicity Detection Kit [LDH], Roche) as described (Gherardini et al Int J Cosmet Sci, 2019, Apr;41(2):164-182). The measurements were performed on supernatants collected on days respective to each culture and normalized to blank.

For the maximum release, HFs were treated with 1% Triton X 100 in vehicle medium at 37° C for 30 minutes in order to induce maximum LDH release into the medium.

### • Alkaline phosphatase activity - Dermal papilla (DP) inductivity

Alkaline phosphatase is an indicator of DP inductivity (Yang, J Dermatol Sci, 2010, Jan;57(1):2-11). Alkaline phosphatase *in situ* activity was measured in the DP.

### • Versican expression - DP inductivity

Versican, a large chondroitin sulfate proteoglycan molecule, is expressed in DP in anagen HFs and diminishes in catagen HFs and its expression correlates with hair inductive properties (Yang, J Dermatol Sci 2010). Versican intensity was analyzed in the DP.

### • CD34 expression - Stem cell progeny

CD34 is a marker expressed by the progeny of HF bulge stem cells. It is expressed in the most external layer of the outer root sheet (ORS) below the isthmus (sub-bulge to suprabulbar regions) (Purba et al., BioEssays, 2014, May;36(5):513-25). CD34 expression and percentage of CD34⁺ cells were analyzed in the suprabulbar ORS.

### • DP fibroblast emigration.

The DP resident fibroblasts are subjected to migration in the transition between anagen and catagen phase (Hawkshaw et al., Journ of Invest Dermatol, 2015, 135(8):2129-2132). In catagen onset, DP fibroblasts emigrate to the dermal cup through the DP stalk, whereas at anagen induction, they immigrate back in the DP. Quantifying the number of DP fibroblasts present in the DP stalk and in the DP (Kloepper et al., Exp Dermatol, 2010, Mar;19(3):305-12) is therefore a direct readout for anagen induction or catagen promotion. K 15 expression - Stem cells.

### • K15 expression - Stem cells.

K15 is a type I cytokeratin expressed in HF bulge stem cells and their progeny (Purba et al., BioEssays, 2014). It is expressed in the bulge, infra-bulge and suprabulbar ORS. Bulge stem cells are identified using K15 expression, cellular morphology and anatomical localization (below the sebaceous glands, at the area where the arrector pili muscle attaches to the HF). K15 expression was analyzed in the bulge basal layer and in the suprabulbar ORS, and proliferation of K15 positive cells was evaluated in combination with Ki67 immunofluorescence staining.

### Statistics

Each dot represents a single hair follicle; Mean± SEM; GraphPad Prism 9; D'Agostino & Pearson omnibus normality test; for group comparisons (# p<0.05, ## p<0.01, ### p<0.001, #### p<0.0001), non-Gaussian distribution (Kruskal-Wallis test, Dunn's multiple comparison test - each group vs vehicle), Gaussian distribution (One-way ANOVA, Dunett's or Holm-Sidak's multiple comparison test - each group vs vehicle); pairwise comparisons(* p<0.05), no Gaussian distribution (Mann-Whitney), Gaussian distribution (t test); ns, not significant. Statistics for non-Gaussian distribution were followed if at least one group did not show a Gaussian distribution within a given group or pairwise comparison.

n= 3-4 donors, individual HFs. Data were first analysed for Gaussian distribution using D'Agostino & Pearson omnibus normality test and outliers were identified and removed using ROUT method (0= 10/o). When data followed Gaussian distribution, we performed one-way ANOVA analysis followed by Holm-Sidak's multiple comparison test - vehicle fixed and Unpaired student's t-test versus vehicle. When data were too small to determine whether the data follow Gaussian distribution or data did not follow Gaussian distribution, we performed Kruskal-Wallis test followed by Dunn's multiple comparison test - vehicle fixed and Mann-Whitney test versus vehicle. Note that the "only anagen" evaluations include only the data of n=3 patients. Donor 1 has no anagen HFs in the vehicle group.

### Results

- All tested concentrations of E4 did not induce HF cytotoxicity (LDH release into the medium) (Fig. 15).
- E4 did not modify hair shaft production (Fig. 16)
- Microscopically, E4 3µM (significantly) and 300nM and 30µM (tendentially) prolonged anagen (Fig. 17).
- E4 3 µM (significantly) and 300nM and 30µM (tendentially) increased hair matrix keratinocytes proliferation, without affecting hair matrix keratinocytes apoptosis (Fig. 18).
- All tested concentrations of E4 tendentially increased versican expression and alkaline phosphatase activity in DP cells (Fig. 19).
- DP fibroblast emigration analysis revealed that all tested concentrations of E4 did not strikingly affect the density of cells in the DP (Fig 20), all concentrations tendentially decreased the density of cells and 300nM and 3µM E4 significantly reduced the total number of cells in the DP stalk (Fig 21), whereas E4 had no significant impact on the density of cells in the inductive dermal cup (Fig 22).
- In the bulge basal layer, E4 3µM significantly decreased the percentage of K15 positive cells, whilst E4 30µM (significantly) and 3µM and 300nM (tendentially) increased their proliferation (Fig 23).
- In the suprabulbar ORS, E4 did not have any effect on the percentage of K15 positive cells, but E4 300nM (significantly) and 3µM (tendentially) decreased their proliferation (Fig 24).
- E4 3µM (significantly) and 300nM and 30µM (tendentially) increased the percentage of CD34 positive cells in the suprabulbar ORS (Fig. 25).

### Discussion and conclusions

The experiments reported here investigate the potential effect of the estrogenic compound E4 on hair cycle, hair matrix keratinocytes, DP inductivity and DP fibroblasts emigration, stem cell maintenance, and generation of progeny in the context of hair growth promotion. The experiments confirmed that the tested concentrations of E4 did not induce HF cytotoxicity (LDH release in to the medium). Most importantly, the data from four individual donors revealed that E4, tendentially (300nM and 30µM) and significantly (3µM), prolonged anagen *ex vivo.* The significant anagen prolonging effect of 3µM E4 was further supported by the decrease in the density of cells in the DP stalk, as DP fibroblasts accumulate in this specific compartment during their emigration from the DP in catagen (Kloepper et al., Exp Dermatol. 2010). The anagen prolonging effect was also supported by a significant increase of hair matrix keratinocytes proliferation after the treatment with 3µM E4.

The data suggested that the anagen prolonging effect of E4 may have been at least in part mediated by stimulation of DP fibroblast inductivity. Indeed, all tested concentrations of E4 seemed to tendentially upregulate alkaline phosphatase activity and versican expression in the DP.

In the bulge, 3µM E4 seemed to tendentially increase the proliferation rate of pluripotent HF stem cells, whereas it significantly reduced the percentage of K15 positive cells. However, the treatment beneficially affected the generation and/or activities of the stem cell progeny in the suprabulbar ORS. Specifically, 3µM E4 tendentially decreased the number of proliferative K15 positive cells and significantly increased the percentage of CD34 positive cells, the latter also further corroborating the anagen prolongation effect of E4 (Purba et al., 2014).

Taken together, these experiments further encourage the exploration of 3µM E4 treatment for hair growth promotion. E4 may have the potential to maintain anagen and possibly prevent HFs from miniaturization. The above experiments are repeated using affected and unaffected terminal and intermediate microdissected full-length HFs from FPHL patients. It is expected to see beneficial effects of the treatment with E4.

### Example 7: A Clinical Study to assess the effect of estetrol treatment in menopausal (peri- and post-menopausal) woman on hair loss

A randomized, double-blind, placebo-controlled, phase II (pilot) study to evaluate the effects of estetrol in postmenopausal women with androgenetic alopecia and to assess the influence on hair parameters is started.

### Study design:

This randomized, double-blind, placebo-controlled, phase II (pilot) study consists of 2 parts, a gynecological and a dermatological part, which take place at 2 different sites specialized for the respective discipline.

The determination of subject eligibility includes a classification of pattern of hair loss by using the Savin Density Scale. Subjects will be randomly allocated to one of the 2 arms and will receive either E4 20 mg as monohydrate or placebo.

Investigations for efficacy of MHT will include evaluations of lipid and glucose metabolism, and bone turnover. Furthermore, the improvement will be assessed globally by the physician using the Clinical Global Impression scale (CGl).Dermatological investigations include quantitative hair measurements, Subject Self-Assessment (SSA), and Investigator's Global Assessment (IGA) of hair growth. Furthermore, a Menopause-Specific Quality of Life [MENQOL] and a dermatological QoL (Dermatology Life Quality Index [DLQI]) questionnaires is answered by the subjects. Safety of estetrol (E4) is assessed throughout the study in all subjects.

### Number of partcipants (planned:

60 participants (Arm 1: 30, Arm 2: 30)
This sample size has been determined for a 5% two-sided type I error and a power of 80% to detect a 20-hairs/cm² difference between groups in Target Area Hair Count (TAHC), assuming a standard deviation of 25 hairs/cm², considering the use of non-parametric approach (Sauerbronn, int J Gynaecol Obstet, 2000, Jan;68(1):35-41).

### Efficacy variables

Efficacy variables for menopause hormone replacement (MHT):
- Evaluation of effects on lipid and glucose metabolism, and bone turnover as assessed by laboratory examination of fasting blood samples
- CGI assessment

Furthermore, a MENQOL questionnaire will be answered by the subjects.

### Dermatological efficacy variables:

Dermatological measurements and assessments are performed at baseline (either at Screening or at the following visit) and at visits (e.g. week 12 and 24; month 4, 8 and 12).

Primary dermatological efficacy variables will be:
- Target Area Hair Count (TAHC),
- Hair Growth Assessment (HGA).

Other variables will be considered as secondary variables.

Hair parameters will be assessed using the following measurements:
**Digital image analysis** from standardized Macro Photography (HairMetrix Phototrichogram, Canfield) for quantitative hair measurements:
Target Area Hair Count (TAHC) and
Target Area Hair Width (TAHW) for non-vellus and vellus/vellus like (miniaturized) hair, separately. A scalp micro dot tattoo is performed at baseline and during one or more of the visits to assist with standardized macro photography.

**Standardized Global Photography** for SSA and IGA using the global photo of the subject's scalp taken at baseline through the Canfield Camera device software and comparing it to the respective real time global photo made instantly available in the Canfield review software installed on the Canfield supplied laptop

**Subject self-assessment of scalp hair growth** will be performed by the following 3 assessments: Hair Growth Assessment (HGA) (Olsen EA, Whiting D, Bergfeld W, Miller J, Hordinsky M, Wanser R, et al. A multicenter, randomized, placebo-controlled, double-blind clinical trial of a novel formulation of 5% Minoxidil topical foam versus placebo in the treatment of androgenetic alopecia in men. J Am Acad Dermatol. 2007;57:767-74)
Scalp hair growth is compared to baseline (Visit X) using the following 7-point scale:
[-3] greatly decreased
[-2] moderately decreased
[-1] slightly decreased
[0] no change
[1] slightly increased
[2] moderately increased
[3] greatly increased for the following statement:
   - "My scalp hair growth compared to Baseline is:...."

### Efficacy variables: Hair Growth Index (HGI):

Hair Growth Index (HGI) (cf. Gubelin Harcha W, Barboza Martinez J, Tsai TF, Katsuoka K, Kawashima M, Tsuboi R, et al. A randomized, active- and placebo-controlled study of the efficacy and safety of different doses of dutasteride versus placebo and finasteride in the treatment of male subjects with androgenetic alopecia. J Am Acad Dermatol. 2014;70:489-98 e3)
Scalp hair growth is compared to baseline (Visit X) on a health outcome questionnaire using the following 7 point scale:
[-3] much less
[-2] moderately less
[-1] slightly less
[0] the same amount
[1] slightly more
[2] moderately more
[3] much more
for the following 3 questions:
- "Since the start of treatment, when I look at my thinning area, I can see...(scalp)".
- "Since the start of treatment, my hair now covers... (scalp)".
- "Since the start of treatment, the appearance (thickness/quality/amount) of the thinning area on my head is...".

Hair Growth Satisfaction Scale (HGSS) (cf. Gubelin Harcha W, Barboza Martinez J, Tsai TF, Katsuoka K, Kawashima M, Tsuboi R, et al. A randomized, active- and placebo-controlled study of the efficacy and safety of different doses of dutasteride versus placebo and finasteride in the treatment of male subjects with androgenetic alopecia. J Am Acad Dermatol. 2014;70:489-98 e3)
Scalp hair growth/appearance is compared to baseline (Visit X) using the following 7-point scale:
[-3] very dissatisfied
[-2] dissatisfied
[-1] somewhat dissatisfied
[0] neutral/neither satisfied nor dissatisfiedf
[1] somewhat satisfied
[2] satisfied
[3] very satisfied
for 5 questions, "How satisfied do you feel about...":
   - The overall appearance of your hair.
   - The appearance of the thinning area(s) within treatment areas on your head.
   - The amount of scalp that can be seen in the treatment areas.
   - The amount of hair in the treatment areas.
   - The growth of hair in the treatment areas.

**Investigator global assessment (IGA) of scalp hair growth** will be performed using the following 7-point scale:
[-3] greatly decreased
[-2] moderately decreased
[-1] slightly decreased
[0] no change
[1] slightly increased
[2] moderately increased
[3] greatly increased

Furthermore, DLQI and Hairdex^{®} (cf. Fischer TW, Schmidt S, Strauss B, Elsner P. Hairdex Ein Instrument zur Untersuchung der krankheitsbezogenen Lebensqualität bei Patienten mit Haarerkrankungen. Hautarzt., 2001 Mar;52 (3):219-27) can be answered by the subjects.

### Safety variables:

- Medical and gynecological history
- Physical examination
- Gynecological examination
- Breast examination
- Dermatological examination of the skin
- Vital signs
- Electrocardiogram (ECG)
- Papanicolaou (PAP) test
- Transvaginal ultrasound (TVUS)
- Mammography
- Laboratory examinations :
   Fasted blood sampling for
   - Hematology/chemistry
   - Lipid/glucose parameters for inclusion

### Blood sampling for:

- Follicle-stimulating hormone (FSH) and thyroid-stimulating hormone (TSH) for inclusion

- Urine pregnancy test
- Prior and concomitant medication will be checked and observed during the entire study period.
- Adverse events (AEs) will be monitored and collected during the entire study period.

### Inclusion criteria:

Subjects will be allocated to treatment if they meet all of the following inclusion criteria:
1. Signed and dated written informed consent form and any required privacy authorization prior to the initiation of any trial procedure, after the nature of the trial has been explained according to local regulatory requirements;
2. Females, ≥40 up to ≤65 years of age at screening;
3. For hysterectomized subjects: documented hysterectomy must have occurred at least 6 weeks prior to the start of screening. Hysterectomy can be total or subtotal (i.e., cervix was not removed);
4. For non-hysterectomized subjects: uterus with bi-layer endometrial thickness ≤ 4 mm on TVUS;
5. Seeking treatment for relief of VMS associated with menopause;
6. Body mass index ≥18.5 kg/m² up to ≤35.0 kg/m²;
7. A mammogram that shows no sign of significant disease performed during screening or within 9 months prior to the start of screening¹;
8. Postmenopausal status defined as any of the following:
   a. For non-hysterectomized subjects:
      - At least 12 months of spontaneous amenorrhea
      - or 6 months of spontaneous amenorrhea with serum FSH levels > 40 mIU/mL ( values obtained after washout of estrogen/progestin containing drugs, if applicable, see exclusion criteria 18 and 20)
      - or 6 weeks postsurgical bilateral oophorectomy2 with or without hysterectomy;
   b. For hysterectomized subjects:
      - serum FSH >40 mlU/mL (values obtained after washout of estrogen/progestin containing drug, if applicable, see exclusion criteria 18 and 20);
      - or at least 6 weeks postsurgical bilateral oophorectomy²;
9. Good physical and mental health, in the judgement of the Investigator as based on medical history, physical and gynecological examination, and clinical assessments performed prior to Visit 1;
10. Able to understand and comply with the protocol requirements, instructions, and protocol-stated restrictions;
11. Able and willing to complete trial daily diaries and questionnaires.
12. Non-smoker of at least one year (including electronic cigarettes)

Further inclusion criteria considering skin condition:
13. Decrease in hair density on the top of the scalp, relative to the sides and back of the scalp, with scalp hair density in involved density area stages n. 2 to n. 6 on the Savin Density Scale.
14. Subject is willing to maintain the same hairstyle, hair length, and hair color throughout the study (implying the same hair coloring interval prior to the approved photos at screening/baseline).
15. Subject agrees to continue her other general hair care products and regimen for the entire study.
16. Subject agrees to maintain same dietary and supplement pattern.

¹Subjects must have a Breast Imaging-Reporting And Data System (BI-RADS) score of 1 or 2 to enroll in the study. An incomplete mammogram result, i.e., BI-RADS 0, is not acceptable and requires further assessment. The site must obtain a copy of the official report for the subject's study file. A digitalized imaging should be obtained if mammography is done as part of this study.

²A report or a statement on letterhead from the subject's physician documenting both ovaries were removed is needed.

### Exclusion criteria:

Subjects will not be allocated to treatment if they meet one of the following exclusion criteria:
1. History of malignancy, with the exception of basal cell or non-metastatic squamous cell carcinoma of the skin if diagnosed more than 1 year prior to the screening visit³;
2. Any clinically significant findings found by the Investigator at the breast examination and/or on mammography suspicious of breast malignancy that would require additional clinical testing to rule out breast cancer (however, simple cysts confirmed by ultrasound are allowed);
3. PAP test with atypical squamous cells undetermined significance (ASC-US) or higher (low-grade squamous intraepithelial lesion [LSIL], atypical squamous cells cannot exclude high-grade squamous intraepithelial lesion [HSIL] [ASC-H], HSIL dysplastic or malignant cells) in sub-totally hysterectomized and non-hysterectomized subjects⁴. Note: ASC-US is allowed if a reflex human papilloma virus (HPV) testing is performed and is negative for high risk oncogene HPV subtype 16 and 18;
4. For non-hysterectomized subjects :
   a. Presence of uterine cancer, endometrial hyperplasia ;
   b. Presence of endometrial polyp(s);
   c. Undiagnosed vaginal bleeding or undiagnosed abnormal uterine bleeding
   d. Endometrial ablation;
   e. Any uterine/endometrial abnormality that in the judgment of the investigator contraindicates the use of estrogen and/or progestin therapy. This includes presence or history of adenomyosis or significant myoma;
5. Systolic blood pressure (BP) higher than 139 mmHg, diastolic BP higher than 89 mmHg⁵ during screening;
6. History of venous or arterial thromboembolic disease (e.g., superficial or deep vein thrombosis, pulmonary embolism, stroke, myocardial infarction, angina pectoris, etc.), or first-degree family history of venous thromboembolism (VTE);
7. History of known acquired of congenital coagulopathy or abnormal coagulation factors, including known thrombophilia's;
8. Laboratory values of fasting glucose above 125 mg/dL and/or glycated hemoglobin above 7.5%⁶;
9. Dyslipoproteinemia (LDL >190 mg/dL and/or triglycerides >300 mg/dL)⁷;
10. Presence or history of gallbladder disease, unless cholecystectomy has been performed;
11. Systemic lupus erythematosus;
12. Any malabsorption disorders including gastric bypass surgery;
13. History of acute liver disease in the preceding 12 months before the start of screening or presence or history of chronic or severe liver disease [alanine transaminase (ALT) or aspartate transaminase (AST) >2x upper limit of normal (ULN), bilirubin >1.5 ULN], or liver tumors;
14. Chronic or current acute renal impairment (estimated glomerular filtration rate <60 mL/min);
15. Positive human immunodeficiency virus (HIV), hepatitis B or C serology;
16. Porphyria;
17. Diagnosis or treatment of major psychiatric disorder (e.g., schizophrenia, bipolar disorder, etc.) in the judgement of the Investigator;
18. Use of estrogen/progestin containing drug(s) up to:
   a. 1 week before screening start for vaginal non-systemic hormonal products (rings, creams, gels);
   b. 4 weeks before screening start for vaginal or transdermal estrogen or estrogen/progestin products with systemic effect ;
   c. 8 weeks before screening start for oral estrogen and/or progestin products and/or selective estrogen receptor modulator therapy;
   d. 8 weeks before screening start for intrauterine progestin therapy;
   e. 3 months before screening start for progestin implants or estrogen alone injectable drug therapy;
   f. 6 months before screening start for estrogen pellet therapy or progestin injectable drug therapy;
19. Use of androgen/ dehydroepiandrosterone (DHEA) containing drugs:
   a. 8 weeks before screening start for oral, topical, vaginal or transdermal androgen;
   b. 6 months before screening start for implantable or injectable androgen therapy;
20. Use of phytoestrogens or black cohosh for treatment of VMS up to 2 weeks before the start of screening;
21. Not willing to stop any hormonal products as described in exclusion criteria 18, 19 and 20 during their participation in the trial;
22. Inadequately treated thyroid dysfunction. Subjects with low or high TSH are allowed if free thyroxine (T4) at screening is within normal range⁸.
23. History or presence of allergy/intolerance to the investigational product or drugs of this class or any component of it, or history of drug or other allergy that, in the opinion of the Investigator contraindicates subject participation;
24. History of alcohol or substance abuse (including marijuana, even if legally allowed) or dependence in the previous 12 months before the start of screening as determined by the Investigator, based on reported observations;
25. Sponsor or CRO employees or employees under the direct supervision of the Investigator and/or involved directly in the trial;
26. Subjects with known or suspected history of a clinically significant systemic disease, unstable medical disorders, life-threatening disease or current malignancies that would pose a risk to the subject in the opinion of the Investigator;
27. Participation in another investigational drug clinical trial within 1 month (30 days) or having received an investigational drug within the last month (30 days) before the start of screening;
28. Is judged by the Investigator to be unsuitable for any reason.

Further exclusion criteria considering impact on hair condition:
29. Subject has any dermatological disorders of the scalp in the target region with the possibility of interfering with the application of the IMP or examination method, such as fungal or bacterial infections, seborrheic dermatitis, psoriasis, eczema, folliculitis, scars, or scalp atrophy.
30. Subject has any skin pathology or condition that, in the investigator's opinion, could interfere with the evaluation of the IMP or requires use of interfering topical, systemic (e.g., uncontrolled thyroid disease, certain genetic disorders that involve hair growth or patterns), or surgical therapy.
31. Subject has a history or any signs of hyperandrogenemia (e.g., excessive facial/pubic/periumbilical hair growth, severe acne, excessive testosterone values in medical history).
32. Subject has current or recent history (within 6 months) of hair weaves, non-breathable wigs or hair bonding.
33. Subject had scalp hair transplants at any time.
34. Subject has a history or active hair loss due to diffuse telogen effluvium, alopecia areata, scarring alopecia, trichotillomania, or conditions/diseases other than AGA.
35. Subject has a current or recent history (within 6 months) of severe dietary or weight changes or has a history of eating disorder(s), any history of bariatric surgery (gastric bypass, gastric sleeve, stomach stapling); macro- or micro-nutrient deficiencies within the last 6 months (i.e., clinically significant iron deficiency, protein deficiency confirmed by laboratory testing) and/or any current diagnosis of malabsorptive disease (i.e., Celiac, Irritable Bowel disease etc.).
36. Subject has used any of the following topical preparations or procedures on the scalp:
   a) Topical scalp treatments for hair growth including Minoxidil, hormone therapy, anti-androgens, or other agents that are known to affect hair growth within 12 weeks prior to baseline (Visit 2).
   b) Topical scalp over-the-counter (OTC) or cosmetic treatments known or reasonably believed to affect hair growth (e.g., brands such as Maxilene, Nioxin, Foltene, etc.) or hair health or hair growth products with saw palmetto, copper, etc. within 4 weeks prior to baseline (Visit 2).
   c) Topical scalp treatments that may have ancillary effect on hair growth including, but not limited to, corticosteroids, pimecrolimus, tacrolimus, and retinoids within 4 weeks prior to baseline (Visit 2).
   d) Scalp procedures (surgical, laser, light, or energy treatments, micro-needling, etc.) within 6 months prior to baseline (Visit 2).
   e) Platelet rich plasma (PRP) procedure on the scalp within 1 year.
37. Subject has used the following systemic medications or procedures:
   a) Beta blockers, cimetidine, diazoxide, or corticosteroids (including intramuscular and intralesional injections) within 12 weeks of Visit 2/baseline. Inhaled, intranasal, or ocular corticosteroids are allowed if use is stable [defined as doses and frequency unchanged for at least 4 weeks prior to baseline (Visit 2)].
   b) Retinoid, isotretinoin, vitamin A intake above 10,000 IU per day, or cyclosporine therapy within 6 months prior to baseline (Visit 2).
   c) Any 5 alpha reductase medications (i.e., Finasteride (Propecia^{®}, etc.), Dutasteride or similar product(s)) within 12 months prior to baseline (Visit 2).
   d) Chemotherapy or cytotoxic agents in the last 5 years.
   e) Radiation of the scalp at any time point .
   f) Anti-androgens within 6 months prior to baseline (Visit 2); Anti-androgens (cyproterone acetate, chlormadinone acetate, etc.) together with contraceptive pills are allowed if use is stable [defined as doses and frequency unchanged for at least 3 months prior to baseline (Visit 2)].
   g) Other systemic therapy, which in the opinion of the investigator, may materially affect the subject's hair or hair growth, including, but not limited to, spironolactone, vitamin (including biotin intake >5mg/day) or homeopathy supplement hair growth or hair health products or other steroid hormones (in any form), including anabolic steroids during the 3 months prior to baseline (Visit 2) or during the study.
³ The exception is only for basal cell carcinoma or non-metastatic squamous cell carcinoma of the skin if either of them was diagnosed more than one year prior to the screening of the subject.
⁴ As indicated by written documentation of a prior test performed within 18 months prior screening or by a test performed at screening.
⁵ BP measurements at screening may be repeated if values are outside the inclusion criteria after sitting for an additional 5 to 10 minutes. The last reading will be used for eligibility. Subjects with mild to moderate hypertension who are controlled on a stable anti hypertension regimen may be enrolled if they meet all inclusion/exclusion criteria. Subjects using methyldopa or clonidine containing antihypertensive medication will not be included.
⁶ Laboratory values of fasting glucose and glycated hemoglobin assessed during the last 6 months, and during washout and screening should be considered.
⁷ Subjects using lipid-lowering therapy should be on a stable dose for at least 1 month before screening.
⁸ Reflex T4 test to be performed at screening only if TSH at screening is outside normal range.

### Statistical methods:

Analyses will be performed on the intention-to-treat and per-protocol population. Categorical factors will be summarized using frequencies and/or percentages, while continuous measures will be described using means and standard deviations.

For normally distributed data, the significance of the within-group change from baseline will be estimated from the Student's t distribution and between-group comparison of the change from baseline will be performed using independent t-tests. Results will be presented as the mean and 95% confidence interval. Equivalent nonparametric methods will be used for non-normally distributed data: Wilcoxon sign test for the within-group change from baseline and Mann-Whitney U test for the between-group comparison. Results will be presented as the median, minimum and maximum.

Analyses will be performed using SAS software (Stat version 15.2; SAS Institute, Cary, NC, USA) with a significance level of 0.05. In this exploratory POC trial, no adjustment for multiplicity of testing will be performed.

## Claims

1. An oral dosage unit comprising an estetrol component in an amount equivalent to about 10 mg to about 25 mg of estetrol for use in preventing or treating female pattern hair loss or female androgenetic alopecia.

2. The oral dosage unit for use according to claim 1, wherein said preventing or treating leads to an improvement in or maintenance of hair texture, quality and/or appearance, preferably wherein said treatment comprises or results in an improvement in target area hair count (TAHC) and/or target area hair width (TAHW).

3. The oral dosage unit for use according to claim 1 or 2, wherein said preventing or treating includes preventing hair loss, reversing hair loss, slowing down hair loss, reducing hair loss, or increasing hair growth.

4. The oral dosage unit for use according to any one of claims 1 to 3, wherein said preventing or treating increases keratinocyte function and/or growth, and connected thereto increases function and/or growth, preferably of epidermal keratinocytes (KCs) such as those residing in the bulge, the outer root sheet (ORS), or the hair bulb.

5. The oral dosage unit for use according to any one of claims 1 to 4, wherein said preventing or treating increases hair follicle mesenchymal fibroblast function and/or growth, and connected thereto increases the function and/or growth of dermal papilla (DP) cells.

6. The oral dosage unit for use according to any one of claims 1 to 5, wherein said preventing or treating implies delaying the onset of or preventing catagen.

7. The oral dosage unit for use according to any one of claims 1 to 6, wherein said preventing or treating implies promoting and/or prolonging anagen.

8. The oral dosage unit for use according to any one of claims 1 to 7, wherein said preventing or treating implies restoring and/or promoting the epithelial-mesenchymal interaction between the hair follicle cells, and/or reducing inflammation and/or oxidative stress in hair follicles.

9. The oral dosage unit for use according to any one of claims 1 to 8, wherein a daily amount equivalent to about 14 mg to about 21 mg of estetrol is administered, preferably an amount of between about 14 mg to about 16 mg or between about 19 mg to about 21 mg of estetrol.

10. The oral dosage unit for use according to any one of claims 1 to 9, wherein said estetrol component is estetrol or an ester thereof, preferably wherein said estetrol component is estetrol monohydrate.

11. The oral dosage unit for use according to any one of claims 1 to 10, wherein a progestogen is present in the oral dosage unit, or wherein a progestogen is used, co-administered or administrated after treatment with estetrol, preferably selected from the group comprising: progesterone, drospirenone, norethisterone, norethisteron-acetate (NETA),norethindrone, dydrogesterone, levonorgestrel (LNG), etonogestrel, norgestrel, nomegestrol, nomegestrol-acetate (NOMAC), trimegestone, nestorone, dydrogesterone, gestodene, desogestrel, norgestimate, cyproterone acetate, dienogest, and chlormadinone, more preferably wherein said progestogen is selected from the group comprising drospirenone, progesterone, or dydrogesterone.

12. The oral dosage unit for use according to claim 11, wherein said progestogen is present in said oral dosage unit, or is used or co-administered or administered in an amount equivalent to from about 1 to about 4 mg, more preferably from about 0.25 to about 4 mg, such as from about 1 to about 3 mg, from about 2.5 to 3.5 mg drospirenone, or wherein said progestogen is administered in an amount equivalent to from about 2.5 to 3.5 mg drospirenone.

13. The oral dosage unit for use according to any one of claims 1 to 12, wherein a further active ingredient suitable for preventing or treating hair loss is present in the oral dosage unit or wherein a further active ingredient suitable for preventing or treating hair loss is co-administered or administered before or after treatment with estetrol.

14. The oral dosage unit for use according to any one of claims 1 to 13, wherein the composition is formulated as an oral dosage unit, preferably wherein the composition is formulated for oral, sublingual, buccal, or sublabial administration.

15. The oral dosage unit for use according to any one of claims 1 to 14, wherein the oral dosage unit is formulated to correspond to a daily dosage unit or respectively is administered as a daily dosage unit.

## Patentansprüche

1. Orale Dosierungseinheit umfassend eine Estetrolkomponente in einer Menge äquivalent zu etwa 10 mg bis etwa 25 mg Estetrol zur Verwendung bei der Vorbeugung oder Behandlung von Haarausfall mit weiblichem Muster oder weiblicher androgenetischer Alopezie.

2. Orale Dosierungseinheit zur Verwendung nach Anspruch 1, wobei die Vorbeugung oder Behandlung zu einer Verbesserung oder Bewahrung von Haartextur, -qualität und/oder -aussehen führt, wobei die Behandlung vorzugsweise eine Verbesserung der Haaranzahl in dem Zielbereich (TAHC) und/oder der Haarbreite in dem Zielbereich (TAHW) umfasst oder dazu führt.

3. Orale Dosierungseinheit zur Verwendung nach Anspruch 1 oder 2, wobei die Vorbeugung oder Behandlung Vorbeugen von Haarausfall, Umkehren von Haarausfall, Verlangsamen von Haarausfall, Verringern von Haarausfall oder Erhöhen von Haarwachstum einschließt.

4. Orale Dosierungseinheit zur Verwendung nach einem der Ansprüche 1 bis 3, wobei die Vorbeugung oder Behandlung Funktion und/oder Wachstum von Keratinozyten erhöht und damit verbunden Funktion und/oder Wachstum, vorzugsweise von epidermalen Keratinozyten (KCs), wie z.B. jenen , die sich in der Ausbuchtung, der äußeren Wurzellage (ORS) oder der Haarwurzel befinden, erhöht.

5. Orale Dosierungseinheit zur Verwendung nach einem der Ansprüche 1 bis 4, wobei die Vorbeugung oder Behandlung Funktion und/oder Wachstum von mesenchymalen Fibroblasten der Haarfollikel erhöht und damit verbunden die Funktion und/oder das Wachstum von dermalen Papillazellen (DP) erhöht.

6. Orale Dosierungseinheit zur Verwendung nach einem der Ansprüche 1 bis 5, wobei die Vorbeugung oder Behandlung Verzögern des Einsetzens oder Verhindern von Katagen einschließt.

7. Orale Dosierungseinheit zur Verwendung nach einem der Ansprüche 1 bis 6, wobei die Vorbeugung oder Behandlung Fördern und/oder Verlängern von Anagen einschließt.

8. Orale Dosierungseinheit zur Verwendung nach einem der Ansprüche 1 bis 7, wobei die Vorbeugung oder Behandlung Wiederherstellen und/oder Fördern der epithelial-mesenchymalen Wechselwirkung zwischen den Haarfollikelzellen und/oder Verringern von Entzündung und/oder oxidativem Stress in Haarfollikeln einschließt.

9. Orale Dosierungseinheit zur Verwendung nach einem der Ansprüche 1 bis 8, wobei eine tägliche Menge äquivalent zu etwa 14 mg bis etwa 21 mg Estetrol verabreicht wird, vorzugsweise eine Menge von zwischen etwa 14 mg und etwa 16 mg oder zwischen etwa 19 mg und etwa 21 mg Estetrol.

10. Orale Dosierungseinheit zur Verwendung nach einem der Ansprüche 1 bis 9, wobei die Estetrolkomponente Estetrol oder ein Ester davon ist, wobei die Estetrolkomponente vorzugsweise Estetrolmonohydrat ist.

11. Orale Dosierungseinheit zur Verwendung nach einem der Ansprüche 1 bis 10, wobei in der oralen Dosierungseinheit ein Progestogen vorhanden ist oder wobei ein Progestogen verwendet wird, gemeinsam mit oder nach Behandlung mit Esterrol verabreicht, vorzugsweise ausgewählt aus der Gruppe umfassend: Progesteron, Drospirenon, Norethisteron, Norethisteronacetat (NETA), Norethindron, Dydrogesteron, Levonorgestrel (LNG), Etnogestrel, Norgestrel, Nomegestrol, Nomegestrolacetat (NOMAC), Trimegeston, Nestoron, Dydrogesteron, Gestoden, Desogestrel, Norgestimat, Cyproteronacetat, Dienogest und Chlormadinon, wobei das Progestogen bevorzugter ausgewählt ist aus der Gruppe umfassend Drospirenon, Progesteron oder Dydrogesteron.

12. Orale Dosierungseinheit zur Verwendung nach Anspruch 11, wobei das Progestogen in einer Menge in der oralen Dosierungseinheit vorhanden ist oder verwendet oder gemeinsam verabreicht oder verabreicht wird, die äquivalent ist zu von etwa 1 bis etwa 4 mg, bevorzugter von etwa 0,25 bis etwa 4 mg, wie z.B. von etwa 1 bis etwa 3 mg, von etwa 2,5 bis 3,5 mg Drospirenon, oder wobei das Progestogen in einer Menge verabreicht wird, die äquivalent ist zu von etwa 2,5 bis 3,5 mg Drospirenon.

13. Orale Dosierungseinheit zur Verwendung nach einem der Ansprüche 1 bis 12, wobei ein weiterer zur Vorbeugung oder Behandlung von Haarausfall geeigneter Wirkstoff in der oralen Dosierungseinheit vorhanden ist oder wobei ein weiterer zur Vorbeugung oder Behandlung von Haarausfall geeigneter Wirkstoff vor oder nach Behandlung mit Estetrol gemeinsam verabreicht oder verabreicht wird.

14. Orale Dosierungseinheit zur Verwendung nach einem der Ansprüche 1 bis 13, wobei die Zusammensetzung als eine orale Dosierungseinheit formuliert ist, wobei die Zusammensetzung vorzugsweise für orale, sublinguale, bukkale oder sublabiale Verabreichung formuliert ist.

15. Orale Dosierungseinheit zur Verwendung nach einem der Ansprüche 1 bis 14, wobei die orale Dosierungseinheit so formuliert ist, dass sie einer täglichen Dosierungseinheit entspricht oder entsprechend als tägliche Dosierungseinheit verabreicht wird.

## Revendications

1. Unité posologique orale comprenant un composant estétrol en une quantité équivalente à environ 10 mg à environ 25 mg d'estétrol, pour utilisation dans la prévention ou le traitement de la chute des cheveux féminins ou de l'alopécie androgénétique féminine.

2. Unité posologique orale pour utilisation selon la revendication 1, dans laquelle ladite prévention ou ledit traitement conduit à une amélioration ou un maintien de la texture, de la qualité et/ou de l'aspect des cheveux, de préférence dans laquelle ledit traitement comprend ou résulte en une amélioration de la numération des cheveux de la zone cible (TAHC) et/ou de la largeur des cheveux de la zone cible (TAHW).

3. Unité posologique orale pour utilisation selon la revendication 1 ou 2, dans laquelle ladite prévention ou ledit traitement comprend la prévention de la chute des cheveux, l'inversion de la chute des cheveux, le ralentissement de la chute des cheveux, la réduction de la chute des cheveux, ou l'augmentation de la croissance des cheveux.

4. Unité posologique orale pour utilisation selon l'une quelconque des revendications 1 à 3, dans laquelle ladite prévention ou ledit traitement augmente la fonction et/ou la croissance des kératinocytes, et reliée à ceux-ci augmente la fonction et/ou la croissance, de préférence des kératinocytes épidermiques (KCS) tels que ceux résidant dans le renflement, la feuille de racine externe (ORS), ou le bulbe capillaire.

5. Unité posologique orale pour utilisation selon l'une quelconque des revendications 1 à 4, dans laquelle ladite prévention ou ledit traitement augmente la fonction et/ou la croissance des fibroblastes mésenchymateux du follicule pileux, et reliée à celle-ci augmente la fonction et/ou la croissance des cellules de la papille dermique (DP).

6. Unité posologique orale pour utilisation selon l'une quelconque des revendications 1 à 5, dans laquelle ladite prévention ou ledit traitement implique le retardement de l'apparition ou la prévention de catagène.

7. Unité posologique orale pour utilisation selon l'une quelconque des revendications 1 à 6, dans laquelle ladite prévention ou ledit traitement implique la promotion et/ou la prolongation de l'anagène.

8. Unité posologique orale pour utilisation selon l'une quelconque des revendications 1 à 7, dans laquelle ladite prévention ou ledit traitement implique la restauration et/ou la promotion de l'interaction épithélio-mésenchymateuse entre les cellules du follicule pileux, et/ou la réduction de l'inflammation et/ou du stress oxydatif dans les follicules pileux.

9. Unité posologique orale pour utilisation selon l'une quelconque des revendications 1 à 8, dans laquelle une quantité journalière équivalente à environ 14 mg à environ 21 mg d'estétrol est administrée, de préférence une quantité comprise entre environ 14 mg et environ 16 mg ou entre environ 19 mg et environ 21 mg d'estétrol.

10. Unité posologique orale pour utilisation selon l'une quelconque des revendications 1 à 9, dans laquelle ledit composant estétrol est l'estétrol ou un ester de celui-ci, de préférence dans laquelle ledit composant estétrol est le monohydrate d'estétrol.

11. Unité posologique orale pour utilisation selon l'une quelconque des revendications 1 à 10, dans laquelle un progestatif est présent dans l'unité posologique orale, ou dans laquelle un progestatif est utilisé, co-administré ou administré après traitement par l'estétrol, de préférence choisi dans le groupe comprenant : progestérone, drospirénone, noréthistérone, acétate de noréthistérone (NETA), noréthindrone, dydrogestérone, lévonorgestrel (LNG), étonogestrel, norgestrel, nomégestrol, acétate de nomégestrol (NOMAC), trimégestone, nestorone, dydrogestérone, gestodène, désogestrel, norgestimate, acétate de cyprotérone, diénogest, et chlormadinone, plus préférablement dans laquelle ledit progestatif est choisi dans le groupe comprenant la drospirénone, la progestérone, ou la dydrogestérone.

12. Unité posologique orale pour utilisation selon la revendication 11, dans laquelle ledit progestatif est présent dans ladite unité posologique orale, ou est utilisé ou co-administré ou administré en une quantité équivalente à environ 1 à environ 4 mg, plus préférablement environ 0,25 à environ 4 mg, par exemple environ 1 à environ 3 mg, environ 2,5 à 3,5 mg de drospirénone. ou dans laquelle ledit progestogène est administré en une quantité équivalente à environ 2,5 à 3,5 mg de drospirénone.

13. Unité posologique orale pour utilisation selon l'une quelconque des revendications 1 à 12, dans laquelle un autre ingrédient actif approprié pour prévenir ou traiter la chute des cheveux est présent dans l'unité posologique orale ou dans laquelle un autre ingrédient actif approprié pour prévenir ou traiter la chute des cheveux est co-administré ou administré avant ou après le traitement par l'estétrol.

14. Unité posologique orale pour utilisation selon l'une quelconque des revendications 1 à 13, dans laquelle la composition est formulée sous la forme d'une unité posologique orale, de préférence dans laquelle la composition est formulée pour une administration orale, sublinguale, buccale, ou sublabiale.

15. Unité posologique orale pour utilisation selon l'une quelconque des revendications 1 à 14, dans laquelle l'unité posologique orale est formulée pour correspondre à une unité posologique quotidienne ou respectivement est administrée sous la forme d'une unité posologique quotidienne.
